Europäisches Patentamt

European Patent Office

Office européen des brevets

(19)

(11) EP 0 905 243 A2

(12) EUROPEAN PATENT APPLICATION

(43) Date of publication:
31.03.1999 Bulletin 1999/13

(51) Int Cl.⁶: **C12N 15/31**, C07K 14/31, C12P 21/00, C07K 16/12, C12Q 1/68, G01N 33/50

(21) Application number: 98306185.4

(22) Date of filing: 03.08.1998

(84) Designated Contracting States:
AT BE CH CY DE DK ES FI FR GB GR IE IT LI LU MC NL PT SE
Designated Extension States:
AL LT LV MK RO SI

(30) Priority: 05.08.1997 US 55387 P

(71) Applicants:
• SMITHKLINE BEECHAM CORPORATION
Philadelphia Pennsylvania 19103 (US)
• SMITHKLINE BEECHAM PLC
Brentford, Middlesex TW8 9EP (GB)

(72) Inventors:
• Lonetto, Michael Athur
King of Prussia, Pennsylvania 19406 (US)
• Warren, Patrick Vernon
King of Prussia, Pennsylvania 19406 (US)
• Burnham, Martin Karl Russel
Brentford, Middlesex, TW8 9EP (GB)

(74) Representative: Mallalieu, Catherine Louise et al
D. Young & Co.,
21 New Fetter Lane
London EC4A 1DA (GB)

(54) **Polynucleotides from Staphylococcus aureus expressed in infected tissue**

(57)     The invention provides novel polypeptides and polynucleotides encoding such polypeptides and methods for producing such polypeptides by recombinant techniques. Also provided are methods for utilizing such polypeptides to screen for antibacterial compounds.

**Annex to the description**

SEQUENCE LISTING

<110> SmithKline Beecham Corporation
  Two New Horizons Court
  Brentford
  Middlesex
  United Kingdom
  TW8 9EP

<120> NOVEL PROKARYOTIC POLYNUCLEOTIDES,
  POLYPEPTIDES AND THEIR USES

<130> GM10059

<150> 60/055,387
<151> 1997-08-05

<160> 48

<170> FastSEQ for Windows Version 3.0

<210> 1
<211> 221
<212> DNA
<213> Staphylococcus aureus

<220>
<221> CDS
<222> (1)...(219)

<400> 1
atg aaa aaa cta tta tta cca tta ata att atg tta tta gtg tta gct      48
Met Lys Lys Leu Leu Leu Pro Leu Ile Ile Met Leu Leu Val Leu Ala
 1     5       10       15

```
gcg tgt ggg aac caa ggt gaa aaa aat aac aaa gct gaa act aaa tct
96
Ala Cys Gly Asn Gln Gly Glu Lys Asn Asn Lys Ala Glu Thr Lys Ser
            20                  25                  30


tat aaa atg gac gat ggc aaa acg gta gat att ccg aaa gac cct aaa
144
Tyr Lys Met Asp Asp Gly Lys Thr Val Asp Ile Pro Lys Asp Pro Lys
            35                  40                  45


cgc att gca gta gtt gcg cca aca tat gct ggt gga ctt aaa aaa tta
192
Arg Ile Ala Val Val Ala Pro Thr Tyr Ala Gly Gly Leu Lys Lys Leu
        50                  55                  60


ggt gca aac att gta gct cgt gcc gaa tt
221
Gly Ala Asn Ile Val Ala Arg Ala Glu
    65                  70



        <210> 2
        <211> 409
        <212> DNA
        <213> Staphylococcus aureus

        <220>
        <221> CDS
        <222> (2)...(406)

        <400> 2
a att cgg cac gag cgt gca ttt att aaa aga gga ctt ttg caa ttt gct
49
  Ile Arg His Glu Arg Ala Phe Ile Lys Arg Gly Leu Leu Gln Phe Ala
    1               5                   10                  15


gat ggt aat gga ttt cag tgt ggc att tgg tat gaa gga acg cta gtt
97
Asp Gly Asn Gly Phe Gln Cys Gly Ile Trp Tyr Glu Gly Thr Leu Val
            20                  25                  30
```

```
ggt gtc atc ggt tta cat gaa att aat cac atg cac aga aaa act tca
145
Gly Val Ile Gly Leu His Glu Ile Asn His Met His Arg Lys Thr Ser
        35                  40                  45

tta ggg tac tat tta tat aaa caa ttt gag ggt cat ggg att atg aca
193
Leu Gly Tyr Tyr Leu Tyr Lys Gln Phe Glu Gly His Gly Ile Met Thr
        50                  55                  60

caa gca ctt gag gca ttg ata aac tat tgt ttc tat aag cct tac ttt
241
Gln Ala Leu Glu Ala Leu Ile Asn Tyr Cys Phe Tyr Lys Pro Tyr Phe
    65                  70                  75                  80

atc tca atc tat att act gcc tct ttt cat ttt tta ata aac tcc atc
289
Ile Ser Ile Tyr Ile Thr Ala Ser Phe His Phe Leu Ile Asn Ser Ile
                85                  90                  95

tat ttt ctc aca cgc ttg gat tta ttt cca cat agt att ttt atc ttt
337
Tyr Phe Leu Thr Arg Leu Asp Leu Phe Pro His Ser Ile Phe Ile Phe
            100                 105                 110

cct tcc cat tta tct act tgg cta ttt att tct tct act ctt cat ctt
385
Pro Ser His Leu Ser Thr Trp Leu Phe Ile Ser Ser Thr Leu His Leu
        115                 120                 125

tct ctt tta att ttt tct ttc taa
409
Ser Leu Leu Ile Phe Ser Phe
        130                 135
```

```
<210> 3
<211> 507
<212> DNA
<213> Staphylococcus aureus
```

25

```
<220>
<221> CDS
<222> (1)...(504)

<400> 3
ggc atc gat aag ctt gat atc gaa ttc ggc acg agc tta tat tta ttc      48
Gly Ile Asp Lys Leu Asp Ile Glu Phe Gly Thr Ser Leu Tyr Leu Phe
 1               5                   10                  15

ttt gac ttt gca ggt tat agt tta ttt gcg ata gca ttt agt tat tta      96
Phe Asp Phe Ala Gly Tyr Ser Leu Phe Ala Ile Ala Phe Ser Tyr Leu
                20                  25                  30

ttc ggt att aaa aca cca cca aac ttc gat aaa cct ttc aaa gcg aaa     144
Phe Gly Ile Lys Thr Pro Pro Asn Phe Asp Lys Pro Phe Lys Ala Lys
            35                  40                  45

aat att aaa gat ttc tgg aat aga tgg cat atg aca tta tca ttc tgg     192
Asn Ile Lys Asp Phe Trp Asn Arg Trp His Met Thr Leu Ser Phe Trp
        50                  55                  60

ttc aga gat tgt att tac atg aga tct tta ttc tac atg tct cgt tta     240
Phe Arg Asp Cys Ile Tyr Met Arg Ser Leu Phe Tyr Met Ser Arg Leu
    65                  70                  75                  80

ata tta ttg aag agt caa ttt gca atg tct aac gtg gca ttc tta atc     288
Ile Leu Leu Lys Ser Gln Phe Ala Met Ser Asn Val Ala Phe Leu Ile
                85                  90                  95

aac ttc ttc ata atg gga att tgg cat ggt atc gaa gtg tat tac att     336
Asn Phe Phe Ile Met Gly Ile Trp His Gly Ile Glu Val Tyr Tyr Ile
                100                 105                 110
```

```
gtt tat ggt tta tac cat gca gca ttg ttt ata ggt tat ggc tat tat
384
Val Tyr Gly Leu Tyr His Ala Ala Leu Phe Ile Gly Tyr Gly Tyr Tyr
        115                 120                 125


gaa cgt tgg cgt aag aaa cat ccg cca cgt tgg caa aat ggt ttc aca
432
Glu Arg Trp Arg Lys Lys His Pro Pro Arg Trp Gln Asn Gly Phe Thr
        130                 135                 140


aca gca ctt aac att gtg att aaa ttc cac ttt gta aca ttt ggc ttt
480
Thr Ala Leu Asn Ile Val Ile Lys Phe His Phe Val Thr Phe Gly Phe
145                 150                 155                 160


tta atc ttc tca cgt aaa ctt ata taa
507
Leu Ile Phe Ser Arg Lys Leu Ile
                    165



        <210> 4
        <211> 401
        <212> DNA
        <213> Staphylococcus aureus

        <220>
        <221> CDS
        <222> (3)...(398)

        <400> 4
aa ttc caa gaa att tat gaa tta aat caa tct ttt aat aaa atg gct
47
    Phe Gln Glu Ile Tyr Glu Leu Asn Gln Ser Phe Asn Lys Met Ala
        1               5                   10                  15


tct gaa att acg caa caa atg aat caa att aaa tcc gaa caa caa gaa
95
Ser Glu Ile Thr Gln Gln Met Asn Gln Ile Lys Ser Glu Gln Gln Glu
                20                  25                  30
```

```
aaa aca gaa ctg att caa aac tta gcc cat gat tta aaa aca cct tta
143
Lys Thr Glu Leu Ile Gln Asn Leu Ala His Asp Leu Lys Thr Pro Leu
            35              40              45


gca agc att att tca tat tct gaa gga cta cgt gat ggt ata atc aca
191
Ala Ser Ile Ile Ser Tyr Ser Glu Gly Leu Arg Asp Gly Ile Ile Thr
        50              55              60


aag gat cat gag att aaa gag tca tac gac ata tta att aaa cac gca
239
Lys Asp His Glu Ile Lys Glu Ser Tyr Asp Ile Leu Ile Lys His Ala
        65              70              75


aac aga tta tca aca tta ttt gat gat atg act cat att atc act tna
287
Asn Arg Leu Ser Thr Leu Phe Asp Asp Met Thr His Ile Ile Thr Xaa
    80              85              90              95


aat aca ggt aaa aca tat ccc cca gaa tta ata caa cta gac caa tta
335
Asn Thr Gly Lys Thr Tyr Pro Pro Glu Leu Ile Gln Leu Asp Gln Leu
                100             105             110


ctt gta tca cca tcg caa cca tat gag gat cgg ttt aan cat gaa aaa
383
Leu Val Ser Pro Ser Gln Pro Tyr Glu Asp Arg Phe Xaa His Glu Lys
                115             120             125


ccg aac att agg agg tga
401
Pro Asn Ile Arg Arg
        130



<210> 5
<211> 444
<212> DNA
<213> Staphylococcus aureus
```

<220>

<221> CDS

<222> (1)...(441)

<400> 5

```
atg gat ggt agt caa tat gga gaa tta caa tat gaa agc ttt aac gct     48
Met Asp Gly Ser Gln Tyr Gly Glu Leu Gln Tyr Glu Ser Phe Asn Ala
  1               5                  10                  15

gct gaa gcg gtt att aca tgc cac ggt gta aat gtt cat cct ggt tca     96
Ala Glu Ala Val Ile Thr Cys His Gly Val Asn Val His Pro Gly Ser
             20                  25                  30

gct aaa aat gca atg gta aac gca ata cgt tta ggt gaa caa ttc gat    144
Ala Lys Asn Ala Met Val Asn Ala Ile Arg Leu Gly Glu Gln Phe Asp
         35                  40                  45

agt ttg cta cct gat agt gaa gtt ccg gag cga aca gaa gga tac gaa    192
Ser Leu Leu Pro Asp Ser Glu Val Pro Glu Arg Thr Glu Gly Tyr Glu
     50                  55                  60

ggc ttt tat cac tta atg aac ttt gaa gga act gtt gaa aaa gca act    240
Gly Phe Tyr His Leu Met Asn Phe Glu Gly Thr Val Glu Lys Ala Thr
 65                  70                  75                  80

ttg caa tac att att cgt gat cat gat aaa aaa caa ttc gaa ttg cgt    288
Leu Gln Tyr Ile Ile Arg Asp His Asp Lys Lys Gln Phe Glu Leu Arg
                 85                  90                  95

aag aaa cgt att tta gaa ata cgt gac gat atc aat gcc cat ttt gaa    336
Lys Lys Arg Ile Leu Glu Ile Arg Asp Asp Ile Asn Ala His Phe Glu
                100                 105                 110
```

```
aat tat cca gtt aaa gtt gat ata tcg gat caa tat ttc aat atg gca
384
Asn Tyr Pro Val Lys Val Asp Ile Ser Asp Gln Tyr Phe Asn Met Ala
        115             120             125

gaa aaa tat tac cat tgc ctc ata tta tcg ata tac cta aac gtg tct
432
Glu Lys Tyr Tyr His Cys Leu Ile Leu Ser Ile Tyr Leu Asn Val Ser
        130             135             140

gtg cca aat tag
444
Val Pro Asn
145


        <210> 6
        <211> 357
        <212> DNA
        <213> Staphylococcus aureus

        <220>
        <221> CDS
        <222> (1)...(354)

        <400> 6
aat tcc cct atc ttt gta ttt gag gaa aat gga agt gta tta ggg ttt
48
Asn Ser Pro Ile Phe Val Phe Glu Glu Asn Gly Ser Val Leu Gly Phe
1               5               10              15

gcg acg ttc ggt tcg ttt aga cct tgg cca gca tat caa tat act atc
96
Ala Thr Phe Gly Ser Phe Arg Pro Trp Pro Ala Tyr Gln Tyr Thr Ile
            20              25              30

gag cat tct att tat gtc gat gct tca gct aga gga aaa ggt atc gct
144
Glu His Ser Ile Tyr Val Asp Ala Ser Ala Arg Gly Lys Gly Ile Ala
            35              40              45
```

```
agt caa tta tta cag cgt tta att gta gag gga aaa gct aaa ggt tat
192
Ser Gln Leu Leu Gln Arg Leu Ile Val Glu Gly Lys Ala Lys Gly Tyr
      50                  55                  60


cgt aca ctt gtt ggt ggt att gat gct tct aat gaa gcg agt att aaa
240
Arg Thr Leu Val Gly Gly Ile Asp Ala Ser Asn Glu Ala Ser Ile Lys
  65                  70                  75                  80


tta cat caa aaa ttc aat ttc aag cat gcc ggc aca ctg acc aat gta
288
Leu His Gln Lys Phe Asn Phe Lys His Ala Gly Thr Leu Thr Asn Val
                  85                  90                  95


ggc tat aaa ttt gat tac tgg gta gat tta gct ttt tat gaa tta gat
336
Gly Tyr Lys Phe Asp Tyr Trp Val Asp Leu Ala Phe Tyr Glu Leu Asp
                 100                 105                 110


tta aaa gat aag agg gtt taa
357
Leu Lys Asp Lys Arg Val
             115
```

```
<210> 7
<211> 500
<212> DNA
<213> Staphylococcus aureus

<220>
<221> CDS
<222> (1)...(498)

<400> 7
att atc gat gaa gaa gat cca gag att gaa tta ctt att ana gaa gtt
48
Ile Ile Asp Glu Glu Asp Pro Glu Ile Glu Leu Leu Ile Xaa Glu Val
  1                   5                  10                  15
```

```
tat cca att nta aat gaa tta gtg aaa gga gaa gtt cga cgt tta att
96
Tyr Pro Ile Xaa Asn Glu Leu Val Lys Gly Glu Val Arg Arg Leu Ile
            20              25              30


gca gat ggn naa att aga cca ggc ggc cgt aaa cct gat gaa atc cgt
144
Ala Asp Xaa Xaa Ile Arg Pro Gly Gly Arg Lys Pro Asp Glu Ile Arg
        35              40              45


cca tta gat tct gaa gtt ggt att tta cca aga acg cat ggt tca ggt
192
Pro Leu Asp Ser Glu Val Gly Ile Leu Pro Arg Thr His Gly Ser Gly
    50              55              60


cta ttt aca cgt ggt cag act caa gca ctt tca gtt tta aca tta ggt
240
Leu Phe Thr Arg Gly Gln Thr Gln Ala Leu Ser Val Leu Thr Leu Gly
65              70              75              80


gct tta ggc gat tat caa tta att gat ggt tta gga cct gaa gaa gaa
288
Ala Leu Gly Asp Tyr Gln Leu Ile Asp Gly Leu Gly Pro Glu Glu Glu
                85              90              95


aaa aga ttc atg cat cat tat aac ttc ccg aac ttt tca gta ggt gaa
336
Lys Arg Phe Met His His Tyr Asn Phe Pro Asn Phe Ser Val Gly Glu
            100             105             110


act ggt cca gta cgt gcg cca ggt cgt cgt gaa att gga cat ggt gcg
384
Thr Gly Pro Val Arg Ala Pro Gly Arg Arg Glu Ile Gly His Gly Ala
        115             120             125


tta ggt gaa aga gca tta aaa tat att att cct gat act gct gat ttc
432
Leu Gly Glu Arg Ala Leu Lys Tyr Ile Ile Pro Asp Thr Ala Asp Phe
        130             135             140
```

```
cca tat aca att cgt att gta agt gag gta ctt gaa tca aac ggt tca
480
Pro Tyr Thr Ile Arg Ile Val Ser Glu Val Leu Glu Ser Asn Gly Ser
145              150              155              160


tca tct caa gcg tcg gaa tt
500
Ser Ser Gln Ala Ser Glu
                 165



    <210> 8
    <211> 498
    <212> DNA
    <213> Staphylococcus aureus

    <220>
    <221> CDS
    <222> (1)...(495)

    <400> 8
aat tcc tcc ata atg ttc ttt tta gat gtg aaa ttg act tta gca gca
48
Asn Ser Ser Ile Met Phe Phe Leu Asp Val Lys Leu Thr Leu Ala Ala
 1               5               10              15


ctg ttt atc ttc cca ttt tac att tta acg gtg tac gtt ttc ttt gga
96
Leu Phe Ile Phe Pro Phe Tyr Ile Leu Thr Val Tyr Val Phe Phe Gly
             20              25              30


aga tta aga aaa ttg acg cgt gag aga tcg caa gca tta gct gag gtt
144
Arg Leu Arg Lys Leu Thr Arg Glu Arg Ser Gln Ala Leu Ala Glu Val
          35              40              45


caa gga ttc ttg cat gaa cgt gtt caa ggt att tca gtc gtt aaa agt
192
Gln Gly Phe Leu His Glu Arg Val Gln Gly Ile Ser Val Val Lys Ser
          50              55              60
```

```
ttt gcg att gaa gac aat gaa gcg aaa aac ttt gat aaa aag aat act
240
Phe Ala Ile Glu Asp Asn Glu Ala Lys Asn Phe Asp Lys Lys Asn Thr
    65              70              75              80


aat ttc tta aca cgt gcg ttg aaa cat aca aga tgg aat gcc tat tcc
288
Asn Phe Leu Thr Arg Ala Leu Lys His Thr Arg Trp Asn Ala Tyr Ser
            85              90              95


ttt ggc gca att aat aca gtt aca gat att gga cca att att gtc atc
336
Phe Gly Ala Ile Asn Thr Val Thr Asp Ile Gly Pro Ile Ile Val Ile
            100             105             110


ggt gtt ggt gca tat ctt gct att tct gga tca atc aca gna ggt aca
384
Gly Val Gly Ala Tyr Leu Ala Ile Ser Gly Ser Ile Thr Xaa Gly Thr
            115             120             125


ctt gca gca ttt gtt gga tac tta gag tta ttg ttc ggg nct tta cgt
432
Leu Ala Ala Phe Val Gly Tyr Leu Glu Leu Leu Phe Gly Xaa Leu Arg
            130             135             140


cgt tta gtc gca tca ttt aca act tta acg cag agt ttg ctt caa tgg
480
Arg Leu Val Ala Ser Phe Thr Thr Leu Thr Gln Ser Leu Leu Gln Trp
145             150             155             160


acc gtg tat tcc aat taa
498
Thr Val Tyr Ser Asn
                165



        <210> 9
        <211> 291
        <212> DNA
        <213> Staphylococcus aureus
```

34

```
<220>
<221> CDS
<222> (1)...(291)

<400> 9
aaa ggt ccg caa cca att caa cgt att aga gac cgc gta tta att gca
48
Lys Gly Pro Gln Pro Ile Gln Arg Ile Arg Asp Arg Val Leu Ile Ala
 1               5                   10                  15

agt agc agc att tca tat gtt gta agt caa tta gag gac aaa ggt tgg
96
Ser Ser Ser Ile Ser Tyr Val Val Ser Gln Leu Glu Asp Lys Gly Trp
                20                  25                  30

att aca cgt gaa aag gat aaa gat gat aaa cgt gta tat atg gct tgt
144
Ile Thr Arg Glu Lys Asp Lys Asp Asp Lys Arg Val Tyr Met Ala Cys
            35                  40                  45

tta act gaa aaa ggt caa agt caa atg gca gat att ttc cct aag cat
192
Leu Thr Glu Lys Gly Gln Ser Gln Met Ala Asp Ile Phe Pro Lys His
        50                  55                  60

gct gag aca tta aca aaa gcg ttt gat gtg tta aca aag gat gaa tta
240
Ala Glu Thr Leu Thr Lys Ala Phe Asp Val Leu Thr Lys Asp Glu Leu
    65                  70                  75                  80

aca atc tta caa caa gcg ttt aag aaa cta agt gca caa tct aca gaa
288
Thr Ile Leu Gln Gln Ala Phe Lys Lys Leu Ser Ala Gln Ser Thr Glu
                85                  90                  95

gtg
291
Val


<210> 10
```

35

```
<211> 618
<212> DNA
<213> Staphylococcus aureus

<220>
<221> CDS
<222> (1)...(618)

<400> 10
agg atg ttg ctc aac atg aat atg cca tta atg ata gat tta aca aat
48
Arg Met Leu Leu Asn Met Asn Met Pro Leu Met Ile Asp Leu Thr Asn
  1               5                  10                  15

aaa aat gtc gtc ata gtt ggt gga ggc gtc gtt gca agt cgt cgg gca
96
Lys Asn Val Val Ile Val Gly Gly Gly Val Val Ala Ser Arg Arg Ala
               20                  25                  30

caa aca tta aat caa tac gtt gaa cat atg acg gtc atc agt ccg aca
144
Gln Thr Leu Asn Gln Tyr Val Glu His Met Thr Val Ile Ser Pro Thr
           35                  40                  45

atc act gaa aaa ctt caa aat atg gta gat aac ggt gtc gtc ata tgg
192
Ile Thr Glu Lys Leu Gln Asn Met Val Asp Asn Gly Val Val Ile Trp
       50                  55                  60

aaa gaa aaa gaa ttt gaa cca agc gat att gta gac gcg tat cta gtt
240
Lys Glu Lys Glu Phe Glu Pro Ser Asp Ile Val Asp Ala Tyr Leu Val
   65                  70                  75                  80

att gca gca acc aat gag cca cgt gtc aat gaa gcg gta aaa caa gcc
288
Ile Ala Ala Thr Asn Glu Pro Arg Val Asn Glu Ala Val Lys Gln Ala
                   85                  90                  95

tta cct gag cat gcc ctt ttt aat aat gtt gga gat gca tca aat ggc
336
```

Leu Pro Glu His Ala Leu Phe Asn Asn Val Gly Asp Ala Ser Asn Gly
        100                 105                 110

aat gtt gta ttt cca agt gca cta cac cgc gac aag cta act atc agt
384
Asn Val Val Phe Pro Ser Ala Leu His Arg Asp Lys Leu Thr Ile Ser
        115                 120                 125

gta tca act gat ggt gcg agt cct aag ttg aca aaa tca att atg gca
432
Val Ser Thr Asp Gly Ala Ser Pro Lys Leu Thr Lys Ser Ile Met Ala
        130                 135                 140

gag ctt gag gcg tta tat cca cca tca tac agt tcg tat atc gac ttt
480
Glu Leu Glu Ala Leu Tyr Pro Pro Ser Tyr Ser Ser Tyr Ile Asp Phe
145                 150                 155                 160

tta tat act tgc cga cag aaa ata aaa gta ctt gat ata aca tat aac
528
Leu Tyr Thr Cys Arg Gln Lys Ile Lys Val Leu Asp Ile Thr Tyr Asn
                165                 170                 175

gaa aag caa cag tta ctg tca caa att gtg tca caa gaa tat tta aat
576
Glu Lys Gln Gln Leu Leu Ser Gln Ile Val Ser Gln Glu Tyr Leu Asn
                180                 185                 190

cat gac aaa caa gct caa ttt tta gcg tgg ttg gat gta aga
618
His Asp Lys Gln Ala Gln Phe Leu Ala Trp Leu Asp Val Arg
                195                 200                 205


        <210> 11
        <211> 74
        <212> PRT
        <213> Staphylococcus aureus

        <400> 11
Met Lys Lys Leu Leu Leu Pro Leu Ile Ile Met Leu Leu Val Leu Ala

```
        1                   5                   10                  15
Ala Cys Gly Asn Gln Gly Glu Lys Asn Asn Lys Ala Glu Thr Lys Ser
                20                  25                  30
Tyr Lys Met Asp Asp Gly Lys Thr Val Asp Ile Pro Lys Asp Pro Lys
            35                  40                  45
Arg Ile Ala Val Val Ala Pro Thr Tyr Ala Gly Gly Leu Lys Lys Leu
        50                  55                  60
Gly Ala Asn Ile Val Ala Arg Ala Glu Xaa
65                  70
```

```
        <210> 12
        <211> 135
        <212> PRT
        <213> Staphylococcus aureus

        <400> 12
Ile Arg His Glu Arg Ala Phe Ile Lys Arg Gly Leu Leu Gln Phe Ala
  1                 5                   10                  15
Asp Gly Asn Gly Phe Gln Cys Gly Ile Trp Tyr Glu Gly Thr Leu Val
                20                  25                  30
Gly Val Ile Gly Leu His Glu Ile Asn His Met His Arg Lys Thr Ser
            35                  40                  45
Leu Gly Tyr Tyr Leu Tyr Lys Gln Phe Glu Gly His Gly Ile Met Thr
        50                  55                  60
Gln Ala Leu Glu Ala Leu Ile Asn Tyr Cys Phe Tyr Lys Pro Tyr Phe
65                  70                  75                  80
Ile Ser Ile Tyr Ile Thr Ala Ser Phe His Phe Leu Ile Asn Ser Ile
                85                  90                  95
Tyr Phe Leu Thr Arg Leu Asp Leu Phe Pro His Ser Ile Phe Ile Phe
            100                 105                 110
Pro Ser His Leu Ser Thr Trp Leu Phe Ile Ser Ser Thr Leu His Leu
            115                 120                 125
Ser Leu Leu Ile Phe Ser Phe
        130                 135
```

```
        <210> 13
        <211> 168
        <212> PRT
        <213> Staphylococcus aureus

        <400> 13
```

```
Gly Ile Asp Lys Leu Asp Ile Glu Phe Gly Thr Ser Leu Tyr Leu Phe
 1               5               10              15
Phe Asp Phe Ala Gly Tyr Ser Leu Phe Ala Ile Ala Phe Ser Tyr Leu
            20              25              30
Phe Gly Ile Lys Thr Pro Pro Asn Phe Asp Lys Pro Phe Lys Ala Lys
        35              40              45
Asn Ile Lys Asp Phe Trp Asn Arg Trp His Met Thr Leu Ser Phe Trp
    50              55              60
Phe Arg Asp Cys Ile Tyr Met Arg Ser Leu Phe Tyr Met Ser Arg Leu
65              70              75              80
Ile Leu Leu Lys Ser Gln Phe Ala Met Ser Asn Val Ala Phe Leu Ile
            85              90              95
Asn Phe Phe Ile Met Gly Ile Trp His Gly Ile Glu Val Tyr Tyr Ile
            100             105             110
Val Tyr Gly Leu Tyr His Ala Ala Leu Phe Ile Gly Tyr Gly Tyr Tyr
            115             120             125
Glu Arg Trp Arg Lys Lys His Pro Pro Arg Trp Gln Asn Gly Phe Thr
    130             135             140
Thr Ala Leu Asn Ile Val Ile Lys Phe His Phe Val Thr Phe Gly Phe
145             150             155             160
Leu Ile Phe Ser Arg Lys Leu Ile
            165


        <210> 14
        <211> 132
        <212> PRT
        <213> Staphylococcus aureus


        <400> 14
Phe Gln Glu Ile Tyr Glu Leu Asn Gln Ser Phe Asn Lys Met Ala Ser
 1               5               10              15
Glu Ile Thr Gln Gln Met Asn Gln Ile Lys Ser Glu Gln Gln Glu Lys
            20              25              30
Thr Glu Leu Ile Gln Asn Leu Ala His Asp Leu Lys Thr Pro Leu Ala
            35              40              45
Ser Ile Ile Ser Tyr Ser Glu Gly Leu Arg Asp Gly Ile Ile Thr Lys
    50              55              60
Asp His Glu Ile Lys Glu Ser Tyr Asp Ile Leu Ile Lys His Ala Asn
65              70              75              80
Arg Leu Ser Thr Leu Phe Asp Asp Met Thr His Ile Ile Thr Xaa Asn
            85              90              95
```

```
Thr Gly Lys Thr Tyr Pro Pro Glu Leu Ile Gln Leu Asp Gln Leu Leu
            100             105             110
Val Ser Pro Ser Gln Pro Tyr Glu Asp Arg Phe Xaa His Glu Lys Pro
        115                 120             125
Asn Ile Arg Arg
    130


        <210> 15
        <211> 147
        <212> PRT
        <213> Staphylococcus aureus


        <400> 15
Met Asp Gly Ser Gln Tyr Gly Glu Leu Gln Tyr Glu Ser Phe Asn Ala
 1               5               10                  15
Ala Glu Ala Val Ile Thr Cys His Gly Val Asn Val His Pro Gly Ser
            20              25                  30
Ala Lys Asn Ala Met Val Asn Ala Ile Arg Leu Gly Glu Gln Phe Asp
        35              40                  45
Ser Leu Leu Pro Asp Ser Glu Val Pro Glu Arg Thr Glu Gly Tyr Glu
        50              55                  60
Gly Phe Tyr His Leu Met Asn Phe Glu Gly Thr Val Glu Lys Ala Thr
65                  70              75                  80
Leu Gln Tyr Ile Ile Arg Asp His Asp Lys Lys Gln Phe Glu Leu Arg
                85              90                  95
Lys Lys Arg Ile Leu Glu Ile Arg Asp Asp Ile Asn Ala His Phe Glu
            100             105             110
Asn Tyr Pro Val Lys Val Asp Ile Ser Asp Gln Tyr Phe Asn Met Ala
        115                 120             125
Glu Lys Tyr Tyr His Cys Leu Ile Leu Ser Ile Tyr Leu Asn Val Ser
        130             135             140
Val Pro Asn
145


        <210> 16
        <211> 118
        <212> PRT
        <213> Staphylococcus aureus


        <400> 16
Asn Ser Pro Ile Phe Val Phe Glu Glu Asn Gly Ser Val Leu Gly Phe
```

```
 1                   5                   10                  15
Ala Thr Phe Gly Ser Phe Arg Pro Trp Pro Ala Tyr Gln Tyr Thr Ile
                20                  25                  30
Glu His Ser Ile Tyr Val Asp Ala Ser Ala Arg Gly Lys Gly Ile Ala
                35                  40                  45
Ser Gln Leu Leu Gln Arg Leu Ile Val Glu Gly Lys Ala Lys Gly Tyr
        50                  55                  60
Arg Thr Leu Val Gly Gly Ile Asp Ala Ser Asn Glu Ala Ser Ile Lys
65                  70                  75                  80
Leu His Gln Lys Phe Asn Phe Lys His Ala Gly Thr Leu Thr Asn Val
                85                  90                  95
Gly Tyr Lys Phe Asp Tyr Trp Val Asp Leu Ala Phe Tyr Glu Leu Asp
                100                 105                 110
Leu Lys Asp Lys Arg Val
        115
```

```
<210> 17
<211> 167
<212> PRT
<213> Staphylococcus aureus
```

```
<400> 17
Ile Ile Asp Glu Glu Asp Pro Glu Ile Glu Leu Leu Ile Xaa Glu Val
 1                   5                   10                  15
Tyr Pro Ile Xaa Asn Glu Leu Val Lys Gly Glu Val Arg Arg Leu Ile
                20                  25                  30
Ala Asp Gly Xaa Ile Arg Pro Gly Gly Arg Lys Pro Asp Glu Ile Arg
                35                  40                  45
Pro Leu Asp Ser Glu Val Gly Ile Leu Pro Arg Thr His Gly Ser Gly
        50                  55                  60
Leu Phe Thr Arg Gly Gln Thr Gln Ala Leu Ser Val Leu Thr Leu Gly
65                  70                  75                  80
Ala Leu Gly Asp Tyr Gln Leu Ile Asp Gly Leu Gly Pro Glu Glu Glu
                85                  90                  95
Lys Arg Phe Met His His Tyr Asn Phe Pro Asn Phe Ser Val Gly Glu
                100                 105                 110
Thr Gly Pro Val Arg Ala Pro Gly Arg Arg Glu Ile Gly His Gly Ala
                115                 120                 125
Leu Gly Glu Arg Ala Leu Lys Tyr Ile Ile Pro Asp Thr Ala Asp Phe
        130                 135                 140
Pro Tyr Thr Ile Arg Ile Val Ser Glu Val Leu Glu Ser Asn Gly Ser
```

145                 150                 155                 160
Ser Ser Gln Ala Ser Glu Xaa
                    165


<210> 18
<211> 165
<212> PRT
<213> Staphylococcus aureus


<400> 18
Asn Ser Ser Ile Met Phe Phe Leu Asp Val Lys Leu Thr Leu Ala Ala
 1               5                  10                  15
Leu Phe Ile Phe Pro Phe Tyr Ile Leu Thr Val Tyr Val Phe Phe Gly
            20                  25                  30
Arg Leu Arg Lys Leu Thr Arg Glu Arg Ser Gln Ala Leu Ala Glu Val
            35                  40                  45
Gln Gly Phe Leu His Glu Arg Val Gln Gly Ile Ser Val Val Lys Ser
            50                  55                  60
Phe Ala Ile Glu Asp Asn Glu Ala Lys Asn Phe Asp Lys Lys Asn Thr
65                  70                  75                  80
Asn Phe Leu Thr Arg Ala Leu Lys His Thr Arg Trp Asn Ala Tyr Ser
                85                  90                  95
Phe Gly Ala Ile Asn Thr Val Thr Asp Ile Gly Pro Ile Ile Val Ile
            100                 105                 110
Gly Val Gly Ala Tyr Leu Ala Ile Ser Gly Ser Ile Thr Xaa Gly Thr
            115                 120                 125
Leu Ala Ala Phe Val Gly Tyr Leu Glu Leu Leu Phe Gly Xaa Leu Arg
            130                 135                 140
Arg Leu Val Ala Ser Phe Thr Thr Leu Thr Gln Ser Leu Leu Gln Trp
145                 150                 155                 160
Thr Val Tyr Ser Asn
                165


<210> 19
<211> 97
<212> PRT
<213> Staphylococcus aureus


<400> 19
Lys Gly Pro Gln Pro Ile Gln Arg Ile Arg Asp Arg Val Leu Ile Ala
 1               5                  10                  15

Ser Ser Ser Ile Ser Tyr Val Val Ser Gln Leu Glu Asp Lys Gly Trp
                20                          25                      30
Ile Thr Arg Glu Lys Asp Lys Asp Asp Lys Arg Val Tyr Met Ala Cys
                35                          40                      45
Leu Thr Glu Lys Gly Gln Ser Gln Met Ala Asp Ile Phe Pro Lys His
                50                          55                      60
Ala Glu Thr Leu Thr Lys Ala Phe Asp Val Leu Thr Lys Asp Glu Leu
65                          70                      75                      80
Thr Ile Leu Gln Gln Ala Phe Lys Lys Leu Ser Ala Gln Ser Thr Glu
                        85                          90                      95
Val


<210> 20
<211> 206
<212> PRT
<213> Staphylococcus aureus


<400> 20
Arg Met Leu Leu Asn Met Asn Met Pro Leu Met Ile Asp Leu Thr Asn
 1                      5                          10                      15
Lys Asn Val Val Ile Val Gly Gly Gly Val Val Ala Ser Arg Arg Ala
                20                          25                      30
Gln Thr Leu Asn Gln Tyr Val Glu His Met Thr Val Ile Ser Pro Thr
                35                          40                      45
Ile Thr Glu Lys Leu Gln Asn Met Val Asp Asn Gly Val Val Ile Trp
                50                          55                      60
Lys Glu Lys Glu Phe Glu Pro Ser Asp Ile Val Asp Ala Tyr Leu Val
65                          70                      75                      80
Ile Ala Ala Thr Asn Glu Pro Arg Val Asn Glu Ala Val Lys Gln Ala
                        85                          90                      95
Leu Pro Glu His Ala Leu Phe Asn Asn Val Gly Asp Ala Ser Asn Gly
                100                         105                     110
Asn Val Val Phe Pro Ser Ala Leu His Arg Asp Lys Leu Thr Ile Ser
                115                         120                     125
Val Ser Thr Asp Gly Ala Ser Pro Lys Leu Thr Lys Ser Ile Met Ala
                130                         135                     140
Glu Leu Glu Ala Leu Tyr Pro Pro Ser Tyr Ser Ser Tyr Ile Asp Phe
145                         150                     155                     160
Leu Tyr Thr Cys Arg Gln Lys Ile Lys Val Leu Asp Ile Thr Tyr Asn
                        165                         170                     175

```
Glu Lys Gln Gln Leu Leu Ser Gln Ile Val Ser Gln Glu Tyr Leu Asn
            180                 185                 190
His Asp Lys Gln Ala Gln Phe Leu Ala Trp Leu Asp Val Arg
        195                 200                 205
```

```
<210> 21
<211> 17
<212> DNA
<213> Staphylococcus aureus

<400> 21
ctattattac cattaat                                          17


<210> 22
<211> 18
<212> DNA
<213> Staphylococcus aureus

<400> 22
aattcggcac gagctaca                                         18


<210> 23
<211> 21
<212> DNA
<213> Staphylococcus aureus

<400> 23
cggcacgagc gtgcatttat t                                     21


<210> 24
<211> 19
<212> DNA
<213> Staphylococcus aureus

<400> 24
ttaaaagaga aagatgaag                                        19
```

<210> 25
<211> 21
<212> DNA
<213> Staphylococcus aureus

<400> 25
ggcatcgata agcttgatat c
21

<210> 26
<211> 19
<212> DNA
<213> Staphylococcus aureus

<400> 26
ttatataagt ttacgtgag
19

<210> 27
<211> 21
<212> DNA
<213> Staphylococcus aureus

<400> 27
ccaagaaatt tatgaattaa a
21

<210> 28
<211> 21
<212> DNA
<213> Staphylococcus aureus

<400> 28
accgatcctc atatggttgc g
21

<210> 29
<211> 17
<212> DNA
<213> Staphylococcus aureus

```
<400> 29
tcaatatgga gaattac          17

<210> 30
<211> 21
<212> DNA
<213> Staphylococcus aureus

<400> 30
aatttggcac agacacgttt a      21

<210> 31
<211> 20
<212> DNA
<213> Staphylococcus aureus

<400> 31
cccctatctt tgtatttgag       20

<210> 32
<211> 19
<212> DNA
<213> Staphylococcus aureus

<400> 32
taaaccctct tatctttta        19

<210> 33
<211> 19
<212> DNA
<213> Staphylococcus aureus

<400> 33
cgatgaagaa gatccagag        19

<210> 34
```

```
<211> 20
<212> DNA
<213> Staphylococcus aureus

<400> 34
caagtacctc acttacaata
20


<210> 35
<211> 20
<212> DNA
<213> Staphylococcus aureus

<400> 35
cctccataat gttctttta
20


<210> 36
<211> 21
<212> DNA
<213> Staphylococcus aureus

<400> 36
attggaatac acggtccatt g
21


<210> 37
<211> 20
<212> DNA
<213> Staphylococcus aureus

<400> 37
aaaggtccgc aaccaattca
20


<210> 38
<211> 21
<212> DNA
<213> Staphylococcus aureus

<400> 38
```

```
cacttctgta gattgtgcac t

21


        <210> 39
        <211> 21
        <212> DNA
        <213> Staphylococcus aureus


        <400> 39
ggatgttgct caacatgaat a

21


        <210> 40
        <211> 21
        <212> DNA
        <213> Staphylococcus aureus


        <400> 40
aaattgagct tgtttgtcat g

21


        <210> 41
        <211> 495
        <212> DNA
        <213> Staphylococcus aureus


        <220>
        <221> CDS
        <222> (1)...(492)


        <400> 41
atg att aga tgc gct aaa aaa gag gat tta aac gct ata tta gcg ata

48
Met Ile Arg Cys Ala Lys Lys Glu Asp Leu Asn Ala Ile Leu Ala Ile
 1               5                   10                  15


tac aat gat gcc att atc aat act aca gct gtt tat act tat gaa cca

96
Tyr Asn Asp Ala Ile Ile Asn Thr Thr Ala Val Tyr Thr Tyr Glu Pro
             20                  25                  30
```

caa acc ata gac gaa cgt gtc gca tgg ttt gaa acg aaa caa cgt aag 144
Gln Thr Ile Asp Glu Arg Val Ala Trp Phe Glu Thr Lys Gln Arg Lys
        35                  40                  45

cat gag cct atc ttt gta ttt gag gaa aat gga agt gta tta ggg ttt 192
His Glu Pro Ile Phe Val Phe Glu Glu Asn Gly Ser Val Leu Gly Phe
        50                  55                  60

gcg acg ttc ggt tcg ttt aga cct tgg cca gca tat caa tat act atc 240
Ala Thr Phe Gly Ser Phe Arg Pro Trp Pro Ala Tyr Gln Tyr Thr Ile
    65                  70                  75                  80

gag cat tct att tat gtc gat gct tca gct aga gga aaa ggt atc gct 288
Glu His Ser Ile Tyr Val Asp Ala Ser Ala Arg Gly Lys Gly Ile Ala
                85                  90                  95

agt caa tta tta cag cgt tta att gta gag gga aaa gct aaa ggt tat 336
Ser Gln Leu Leu Gln Arg Leu Ile Val Glu Gly Lys Ala Lys Gly Tyr
            100                 105                 110

cgt aca ctt gtt ggt ggt att gat gct tct aat gaa gcg agt att aaa 384
Arg Thr Leu Val Gly Gly Ile Asp Ala Ser Asn Glu Ala Ser Ile Lys
            115                 120                 125

tta cat caa aaa ttc aat ttc aag cat gcc ggc aca ctg acc aat gta 432
Leu His Gln Lys Phe Asn Phe Lys His Ala Gly Thr Leu Thr Asn Val
        130                 135                 140

ggc tat aaa ttt gat tac tgg gta gat tta gct ttt tat gaa tta gat 480
Gly Tyr Lys Phe Asp Tyr Trp Val Asp Leu Ala Phe Tyr Glu Leu Asp
145                 150                 155                 160

```
tta aaa gat aag taa
495
Leu Lys Asp Lys


        <210> 42
        <211> 2097
        <212> DNA
        <213> Staphylococcus aureus

        <220>
        <221> CDS
        <222> (1)...(2094)

        <400> 42
 atg tct caa gaa aag aaa gtt ttt aaa act gaa tgg gca gga aga tct
 48
 Met Ser Gln Glu Lys Lys Val Phe Lys Thr Glu Trp Ala Gly Arg Ser
  1               5                  10                  15

 tta acg att gaa aca ggg caa tta gct aaa caa gca aat ggc gct gta
 96
 Leu Thr Ile Glu Thr Gly Gln Leu Ala Lys Gln Ala Asn Gly Ala Val
                20                  25                  30

 ttg gtt cgt tat gga gat aca gtc gtg tta tcg acg gca act gca tca
 144
 Leu Val Arg Tyr Gly Asp Thr Val Val Leu Ser Thr Ala Thr Ala Ser
             35                  40                  45

 aaa gaa cct cgt gat gga gat ttc ttc cca tta aca gtg aac tat gaa
 192
 Lys Glu Pro Arg Asp Gly Asp Phe Phe Pro Leu Thr Val Asn Tyr Glu
         50                  55                  60

 gaa aaa atg tac gct gcg ggt aaa att cct ggt gga ttt aaa aag aga
 240
 Glu Lys Met Tyr Ala Ala Gly Lys Ile Pro Gly Gly Phe Lys Lys Arg
     65                  70                  75                  80
```

```
gaa gga cgt cct ggt gac gat gca aca tta act gcg cga tta att gat
288
Glu Gly Arg Pro Gly Asp Asp Ala Thr Leu Thr Ala Arg Leu Ile Asp
                85                  90                  95


aga cca att aga cct tta ttc cct aaa gga tat aag cat gat gtt caa
336
Arg Pro Ile Arg Pro Leu Phe Pro Lys Gly Tyr Lys His Asp Val Gln
            100                 105                 110


att atg aac atg gta tta agt gca gat cct gat tgt tca cca caa atg
384
Ile Met Asn Met Val Leu Ser Ala Asp Pro Asp Cys Ser Pro Gln Met
            115                 120                 125


gct gca atg att ggt tca tct atg gcg ctt agt gtg tcg gat att cca
432
Ala Ala Met Ile Gly Ser Ser Met Ala Leu Ser Val Ser Asp Ile Pro
        130                 135                 140


ttc caa ggg cca atc gcc ggt gta aat gtg ggt tat att gac ggt aaa
480
Phe Gln Gly Pro Ile Ala Gly Val Asn Val Gly Tyr Ile Asp Gly Lys
145                 150                 155                 160


tat atc att aac cca aca gta gaa gaa aaa gaa gtt tct cgt tta gac
528
Tyr Ile Ile Asn Pro Thr Val Glu Glu Lys Glu Val Ser Arg Leu Asp
                165                 170                 175


ctt gaa gta gct ggt cat aaa gat gca gta aac atg gta gag gca ggc
576
Leu Glu Val Ala Gly His Lys Asp Ala Val Asn Met Val Glu Ala Gly
            180                 185                 190


gct agt gag att act gaa caa gaa atg tta gag gcg att ttc ttt ggt
624
Ala Ser Glu Ile Thr Glu Gln Glu Met Leu Glu Ala Ile Phe Phe Gly
            195                 200                 205
```

```
cat gaa gag att caa cgt tta gtt gat ttc caa caa caa atc gtc gac
672
His Glu Glu Ile Gln Arg Leu Val Asp Phe Gln Gln Gln Ile Val Asp
    210             215             220

cac att caa cct gtt aaa caa gaa ttt att cca gca gag cgt gat gaa
720
His Ile Gln Pro Val Lys Gln Glu Phe Ile Pro Ala Glu Arg Asp Glu
225             230             235             240

gcg cta gtt gaa cgt ata aaa tct tta acc gaa gaa aaa gga ctt aaa
768
Ala Leu Val Glu Arg Ile Lys Ser Leu Thr Glu Glu Lys Gly Leu Lys
                245             250             255

gaa aca gtt tta aca ttt gat aaa caa caa cga gat gaa aat ctt gat
816
Glu Thr Val Leu Thr Phe Asp Lys Gln Gln Arg Asp Glu Asn Leu Asp
                260             265             270

aac tta aaa gaa gaa atc gtc aat gaa ttt atc gat gaa gaa gat cca
864
Asn Leu Lys Glu Glu Ile Val Asn Glu Phe Ile Asp Glu Glu Asp Pro
            275             280             285

gag aat gaa tta ctt att aaa gaa gtt tat gca att tta aat gaa tta
912
Glu Asn Glu Leu Leu Ile Lys Glu Val Tyr Ala Ile Leu Asn Glu Leu
        290             295             300

gtg aaa gaa gaa gtt cga cgt tta att gca gat gaa aaa att aga cca
960
Val Lys Glu Glu Val Arg Arg Leu Ile Ala Asp Glu Lys Ile Arg Pro
305             310             315             320

gac ggc cgt aaa cct gat gaa atc cgt cca tta gat tct gaa gtt ggt
1008
Asp Gly Arg Lys Pro Asp Glu Ile Arg Pro Leu Asp Ser Glu Val Gly
                325             330             335
```

att tta cct aga acg cat ggt tca ggt cta ttt aca cgt ggt cag act
1056
Ile Leu Pro Arg Thr His Gly Ser Gly Leu Phe Thr Arg Gly Gln Thr
            340                 345                 350

caa gca ctt tca gtt tta acg tta ggt gct tta ggc gat tat caa tta
1104
Gln Ala Leu Ser Val Leu Thr Leu Gly Ala Leu Gly Asp Tyr Gln Leu
            355                 360                 365

att gat ggt tta gga cct gaa gaa gaa aaa aga ttc atg cat cat tat
1152
Ile Asp Gly Leu Gly Pro Glu Glu Glu Lys Arg Phe Met His His Tyr
            370                 375                 380

aac ttc ccg aat ttt tca gta ggt gaa act ggt cca gta cgt gcg cca
1200
Asn Phe Pro Asn Phe Ser Val Gly Glu Thr Gly Pro Val Arg Ala Pro
385                 390                 395                 400

ggt cgt cgt gaa att gga cat ggt gcg tta ggt gaa aga gca tta aaa
1248
Gly Arg Arg Glu Ile Gly His Gly Ala Leu Gly Glu Arg Ala Leu Lys
                405                 410                 415

tat att att cct gat act gct gat ttc cca tat aca att cgt att gta
1296
Tyr Ile Ile Pro Asp Thr Ala Asp Phe Pro Tyr Thr Ile Arg Ile Val
            420                 425                 430

agt gag gta ctt gaa tca aac ggt tca tca tct caa gcg tca att tgt
1344
Ser Glu Val Leu Glu Ser Asn Gly Ser Ser Ser Gln Ala Ser Ile Cys
            435                 440                 445

gga tca aca tta gct tta atg gat gcg ggc gta ccg att aaa gca cca
1392
Gly Ser Thr Leu Ala Leu Met Asp Ala Gly Val Pro Ile Lys Ala Pro
            450                 455                 460

```
gtt gct ggt att gct atg ggc ctt gtt aca cgt gaa gat agc tat acg
1440
Val Ala Gly Ile Ala Met Gly Leu Val Thr Arg Glu Asp Ser Tyr Thr
465                 470             475                 480


att tta act gat atc caa ggt atg gaa gat gca tta ggt gat atg gac
1488
Ile Leu Thr Asp Ile Gln Gly Met Glu Asp Ala Leu Gly Asp Met Asp
                485             490             495


ttt aaa gtc gct ggt act aaa gaa ggt att aca gca atc caa atg gat
1536
Phe Lys Val Ala Gly Thr Lys Glu Gly Ile Thr Ala Ile Gln Met Asp
            500             505             510


att aaa att gac ggt tta aca cgt gaa att atc gaa gag gct cta gaa
1584
Ile Lys Ile Asp Gly Leu Thr Arg Glu Ile Ile Glu Glu Ala Leu Glu
            515             520             525


caa gcg aga cgt ggt cgt tta gaa ata atg aat cat atg tta caa aca
1632
Gln Ala Arg Arg Gly Arg Leu Glu Ile Met Asn His Met Leu Gln Thr
        530             535             540


att gat caa cca cgt act gaa tta agt gct tac gcg cca aaa gtt gta
1680
Ile Asp Gln Pro Arg Thr Glu Leu Ser Ala Tyr Ala Pro Lys Val Val
545             550             555             560


act atg aca att aaa cca gat aag att aga gat gtt atc gga cct ggt
1728
Thr Met Thr Ile Lys Pro Asp Lys Ile Arg Asp Val Ile Gly Pro Gly
            565             570             575


ggt aaa aaa atc aac gaa att att gat gaa aca ggc gtt aaa tta gat
1776
Gly Lys Lys Ile Asn Glu Ile Ile Asp Glu Thr Gly Val Lys Leu Asp
            580             585             590
```

EP 0 905 243 A2

att gaa caa gat ggt act atc ttt att ggt gcc gtt gat caa gct atg
1824
Ile Glu Gln Asp Gly Thr Ile Phe Ile Gly Ala Val Asp Gln Ala Met
          595               600               605

ata aat cgt gct cgt gaa atc att gag gaa att aca cgt gaa gcg gaa
1872
Ile Asn Arg Ala Arg Glu Ile Ile Glu Glu Ile Thr Arg Glu Ala Glu
          610               615               620

gta ggt caa act tat caa gcc act gtt aaa cgt att gaa aaa tac ggt
1920
Val Gly Gln Thr Tyr Gln Ala Thr Val Lys Arg Ile Glu Lys Tyr Gly
625               630               635               640

gcg ttt gta ggc cta ttc ccg ggt aaa gat gcg ttg ctt cac att tca
1968
Ala Phe Val Gly Leu Phe Pro Gly Lys Asp Ala Leu Leu His Ile Ser
               645               650               655

caa att tca aaa aat aga att gaa aaa gtg gaa gat gta tta aaa atc
2016
Gln Ile Ser Lys Asn Arg Ile Glu Lys Val Glu Asp Val Leu Lys Ile
          660               665               670

ggt gac aca att gaa gtt aag att act gaa att gat aaa caa ggt cga
2064
Gly Asp Thr Ile Glu Val Lys Ile Thr Glu Ile Asp Lys Gln Gly Arg
          675               680               685

gta aat gca tca cac aga gca tta gaa gaa taa
2097
Val Asn Ala Ser His Arg Ala Leu Glu Glu
          690               695

<210> 43
<211> 1737
<212> DNA
<213> Staphylococcus aureus

55

```
<220>
<221> CDS
<222> (1)...(1734)

<400> 43
atg att aaa cga tat ttg caa ttt gtt aag cca tac aaa tat cgt att    48
Met Ile Lys Arg Tyr Leu Gln Phe Val Lys Pro Tyr Lys Tyr Arg Ile
 1               5                  10                  15

ttt gca acg att att gtt ggg ata att aag ttt ggt ata cca atg ctt    96
Phe Ala Thr Ile Ile Val Gly Ile Ile Lys Phe Gly Ile Pro Met Leu
                20                  25                  30

ata cca ctt tta att aaa tat gca ata gat ggc gtg att aat aac cac   144
Ile Pro Leu Leu Ile Lys Tyr Ala Ile Asp Gly Val Ile Asn Asn His
            35                  40                  45

gca cta acg act gat gaa aaa gtt cat cat tta act att gca att ggt   192
Ala Leu Thr Thr Asp Glu Lys Val His His Leu Thr Ile Ala Ile Gly
        50                  55                  60

atc gca tta ttt att ttt gta ata gtg aga cca cca att gaa ttt ata   240
Ile Ala Leu Phe Ile Phe Val Ile Val Arg Pro Pro Ile Glu Phe Ile
    65                  70                  75                  80

cgt caa tat ttg gcg caa tgg aca agt aat aaa ata ttg tat gat ata   288
Arg Gln Tyr Leu Ala Gln Trp Thr Ser Asn Lys Ile Leu Tyr Asp Ile
                    85                  90                  95

cgt aaa aag tta tat aac cat tta caa gct cta agt gcg aga ttt tat   336
Arg Lys Lys Leu Tyr Asn His Leu Gln Ala Leu Ser Ala Arg Phe Tyr
                100                 105                 110
```

gct aat aat caa gta ggt caa gta ata tct aga gtg att aat gat gtt
384
Ala Asn Asn Gln Val Gly Gln Val Ile Ser Arg Val Ile Asn Asp Val
        115           120           125

gaa caa aca aaa gat ttc att tta acc ggg tta atg aat att tgg tta
432
Glu Gln Thr Lys Asp Phe Ile Leu Thr Gly Leu Met Asn Ile Trp Leu
        130           135           140

gat tgt ata aca att att att gca cta tcc ata atg ttc ttt tta gat
480
Asp Cys Ile Thr Ile Ile Ile Ala Leu Ser Ile Met Phe Phe Leu Asp
145           150           155           160

gtg aaa ttg act tta gca gca ctg ttt atc ttc cca ttt tac att tta
528
Val Lys Leu Thr Leu Ala Ala Leu Phe Ile Phe Pro Phe Tyr Ile Leu
        165           170           175

acg gtg tac gtt ttc ttt gga aga tta aga aaa ttg acg cgt gaa aga
576
Thr Val Tyr Val Phe Phe Gly Arg Leu Arg Lys Leu Thr Arg Glu Arg
        180           185           190

tct caa gca tta gct gag gtt caa gga ttc ttg cat gaa cgt gtt caa
624
Ser Gln Ala Leu Ala Glu Val Gln Gly Phe Leu His Glu Arg Val Gln
        195           200           205

ggt att tca gtc gtt aaa agt ttt gcg att gaa gac aat gaa gcg aaa
672
Gly Ile Ser Val Val Lys Ser Phe Ala Ile Glu Asp Asn Glu Ala Lys
        210           215           220

aac ttt gat aaa aag aac act aat ttc tta aca cgt gcg ttg aaa cat
720
Asn Phe Asp Lys Lys Asn Thr Asn Phe Leu Thr Arg Ala Leu Lys His
225           230           235           240

aca aga tgg aat gcc tat tcc ttt gcc gca att aat aca gtt aca gat
768
Thr Arg Trp Asn Ala Tyr Ser Phe Ala Ala Ile Asn Thr Val Thr Asp
                245                 250                 255

att gga cca ata att gtc atc ggt gtt ggt gca tat ctt gct att tct
816
Ile Gly Pro Ile Ile Val Ile Gly Val Gly Ala Tyr Leu Ala Ile Ser
            260                 265                 270

gga tca atc aca gta ggt aca ctt gca gca ttt gtt gga tac ttg gag
864
Gly Ser Ile Thr Val Gly Thr Leu Ala Ala Phe Val Gly Tyr Leu Glu
            275                 280                 285

tta ttg ttt ggg cct tta cgt cgt tta gtc gca tca ttt aca act tta
912
Leu Leu Phe Gly Pro Leu Arg Arg Leu Val Ala Ser Phe Thr Thr Leu
        290                 295                 300

acg caa agt ttt gct tca atg gac cgt gta ttc caa tta att gac gaa
960
Thr Gln Ser Phe Ala Ser Met Asp Arg Val Phe Gln Leu Ile Asp Glu
305                 310                 315                 320

gat tat gac att aaa aat ggt gtt ggt gct caa cct att gaa att aaa
1008
Asp Tyr Asp Ile Lys Asn Gly Val Gly Ala Gln Pro Ile Glu Ile Lys
                325                 330                 335

caa ggt cgt att gat att gat cat gtt agt ttt caa tat aac gat aac
1056
Gln Gly Arg Ile Asp Ile Asp His Val Ser Phe Gln Tyr Asn Asp Asn
            340                 345                 350

gaa gct cca att tta aaa gat att aat ttg agt att gaa aaa gga gaa
1104
Glu Ala Pro Ile Leu Lys Asp Ile Asn Leu Ser Ile Glu Lys Gly Glu
            355                 360                 365

```
aca gtt gct ttc gta ggt atg agt ggt ggt ggt aaa tca aca tta att
1152
Thr Val Ala Phe Val Gly Met Ser Gly Gly Gly Lys Ser Thr Leu Ile
    370                 375                 380


aac tta ata ccg aga ttt tac gat gta act tct ggg caa att tta ata
1200
Asn Leu Ile Pro Arg Phe Tyr Asp Val Thr Ser Gly Gln Ile Leu Ile
385                 390                 395                 400


gat ggt cac aac att aaa gat ttt tta acg gga agt tta aga aat caa
1248
Asp Gly His Asn Ile Lys Asp Phe Leu Thr Gly Ser Leu Arg Asn Gln
                405                 410                 415


ata gga ttg gtg caa cag gat aat att tta ttc tcc gat aca gtt aag
1296
Ile Gly Leu Val Gln Gln Asp Asn Ile Leu Phe Ser Asp Thr Val Lys
                420                 425                 430


gaa aat att tta ctt ggt cgt cca aca gca aca gat gaa gaa gta gtt
1344
Glu Asn Ile Leu Leu Gly Arg Pro Thr Ala Thr Asp Glu Glu Val Val
                435                 440                 445


gaa gcg gcg aaa atg gct aat gca cat gac ttt att atg act ttg cca
1392
Glu Ala Ala Lys Met Ala Asn Ala His Asp Phe Ile Met Thr Leu Pro
    450                 455                 460


cag gga tat gac act gaa gta ggt gaa cga ggt gtt aaa tta tca ggt
1440
Gln Gly Tyr Asp Thr Glu Val Gly Glu Arg Gly Val Lys Leu Ser Gly
465                 470                 475                 480


ggt caa aaa caa aga tta tcg att gct aga ata ttt tta aat aat ccg
1488
Gly Gln Lys Gln Arg Leu Ser Ile Ala Arg Ile Phe Leu Asn Asn Pro
                485                 490                 495
```

```
cca att ctt atc ctg gat gaa gca aca agt gca ctt gat tta gaa agt
1536
Pro Ile Leu Ile Leu Asp Glu Ala Thr Ser Ala Leu Asp Leu Glu Ser
        500                 505                 510


gaa tcc att att caa gaa gca tta gat gtg ttg agt aaa gat cga acg
1584
Glu Ser Ile Ile Gln Glu Ala Leu Asp Val Leu Ser Lys Asp Arg Thr
        515                 520                 525


aca ctt atc gta gcg cat cgc ttg tct act att aca cat gct gac aaa
1632
Thr Leu Ile Val Ala His Arg Leu Ser Thr Ile Thr His Ala Asp Lys
    530                 535                 540


att gtc gta att gaa aat ggg cat att gtt gaa aca ggt acg cat cgt
1680
Ile Val Val Ile Glu Asn Gly His Ile Val Glu Thr Gly Thr His Arg
545                 550                 555                 560


gaa ttg att gca aaa caa ggt gct tac gag cat tta tat agc att caa
1728
Glu Leu Ile Ala Lys Gln Gly Ala Tyr Glu His Leu Tyr Ser Ile Gln
            565                 570                 575


aac tta taa
1737
Asn Leu
```

```
<210> 44
<211> 435
<212> DNA
<213> Staphylococcus aureus

<220>
<221> CDS
<222> (1)...(432)

<400> 44
```

60

```
atg gat cga acg aaa caa tcg ctc aat gtc ttt gtc gga atg aat agg
48
Met Asp Arg Thr Lys Gln Ser Leu Asn Val Phe Val Gly Met Asn Arg
  1               5                  10                  15


gcg tta gac aca tta gag caa att aca aaa gaa gac gta aag cga tat
96
Ala Leu Asp Thr Leu Glu Gln Ile Thr Lys Glu Asp Val Lys Arg Tyr
              20                  25                  30


ggc tta aat att act gaa ttt gca gtg cta gag ttg ctt tat aat aaa
144
Gly Leu Asn Ile Thr Glu Phe Ala Val Leu Glu Leu Leu Tyr Asn Lys
             35                  40                  45


ggt ccg caa cca att caa cgt att aga gac cgc gta tta att gca agt
192
Gly Pro Gln Pro Ile Gln Arg Ile Arg Asp Arg Val Leu Ile Ala Ser
         50                  55                  60


agc agc att tca tat gtt gta agt caa tta gag gac aaa ggt tgg att
240
Ser Ser Ile Ser Tyr Val Val Ser Gln Leu Glu Asp Lys Gly Trp Ile
 65                  70                  75                  80


aca cgt gaa aag gat aaa gat gat aaa cgt gta tat atg gct tgt tta
288
Thr Arg Glu Lys Asp Lys Asp Asp Lys Arg Val Tyr Met Ala Cys Leu
                 85                  90                  95


act gaa aaa ggt caa agt caa atg gca gat att ttc cct aag cat gct
336
Thr Glu Lys Gly Gln Ser Gln Met Ala Asp Ile Phe Pro Lys His Ala
                100                 105                 110


gag aca tta aca aaa gcg ttt gat gtg tta aca aag gat gaa tta aca
384
Glu Thr Leu Thr Lys Ala Phe Asp Val Leu Thr Lys Asp Glu Leu Thr
              115                 120                 125
```

```
atc tta caa caa gcg ttt aag aaa cta agt gca caa tct aca gaa gtg
432
Ile Leu Gln Gln Ala Phe Lys Lys Leu Ser Ala Gln Ser Thr Glu Val
    130                 135                 140

taa
435
```

```
<210> 45
<211> 164
<212> PRT
<213> Staphylococcus aureus

<400> 45
Met Ile Arg Cys Ala Lys Lys Glu Asp Leu Asn Ala Ile Leu Ala Ile
  1               5                  10                  15
Tyr Asn Asp Ala Ile Ile Asn Thr Thr Ala Val Tyr Thr Tyr Glu Pro
            20                  25                  30
Gln Thr Ile Asp Glu Arg Val Ala Trp Phe Glu Thr Lys Gln Arg Lys
          35                  40                  45
His Glu Pro Ile Phe Val Phe Glu Glu Asn Gly Ser Val Leu Gly Phe
        50                  55                  60
Ala Thr Phe Gly Ser Phe Arg Pro Trp Pro Ala Tyr Gln Tyr Thr Ile
65                  70                  75                  80
Glu His Ser Ile Tyr Val Asp Ala Ser Ala Arg Gly Lys Gly Ile Ala
              85                  90                  95
Ser Gln Leu Leu Gln Arg Leu Ile Val Glu Gly Lys Ala Lys Gly Tyr
            100                 105                 110
Arg Thr Leu Val Gly Gly Ile Asp Ala Ser Asn Glu Ala Ser Ile Lys
          115                 120                 125
Leu His Gln Lys Phe Asn Phe Lys His Ala Gly Thr Leu Thr Asn Val
      130                 135                 140
Gly Tyr Lys Phe Asp Tyr Trp Val Asp Leu Ala Phe Tyr Glu Leu Asp
145                 150                 155                 160
Leu Lys Asp Lys
```

```
<210> 46
<211> 698
<212> PRT
<213> Staphylococcus aureus
```

62

```
<400> 46

Met Ser Gln Glu Lys Lys Val Phe Lys Thr Glu Trp Ala Gly Arg Ser
1               5                   10                  15
Leu Thr Ile Glu Thr Gly Gln Leu Ala Lys Gln Ala Asn Gly Ala Val
            20                  25                  30
Leu Val Arg Tyr Gly Asp Thr Val Val Leu Ser Thr Ala Thr Ala Ser
            35                  40                  45
Lys Glu Pro Arg Asp Gly Asp Phe Phe Pro Leu Thr Val Asn Tyr Glu
        50                  55                  60
Glu Lys Met Tyr Ala Ala Gly Lys Ile Pro Gly Gly Phe Lys Lys Arg
65                  70                  75                  80
Glu Gly Arg Pro Gly Asp Asp Ala Thr Leu Thr Ala Arg Leu Ile Asp
                85                  90                  95
Arg Pro Ile Arg Pro Leu Phe Pro Lys Gly Tyr Lys His Asp Val Gln
            100                 105                 110
Ile Met Asn Met Val Leu Ser Ala Asp Pro Asp Cys Ser Pro Gln Met
            115                 120                 125
Ala Ala Met Ile Gly Ser Ser Met Ala Leu Ser Val Ser Asp Ile Pro
        130                 135                 140
Phe Gln Gly Pro Ile Ala Gly Val Asn Val Gly Tyr Ile Asp Gly Lys
145                 150                 155                 160
Tyr Ile Ile Asn Pro Thr Val Glu Glu Lys Glu Val Ser Arg Leu Asp
                165                 170                 175
Leu Glu Val Ala Gly His Lys Asp Ala Val Asn Met Val Glu Ala Gly
                180                 185                 190
Ala Ser Glu Ile Thr Glu Gln Glu Met Leu Glu Ala Ile Phe Phe Gly
        195                 200                 205
His Glu Glu Ile Gln Arg Leu Val Asp Phe Gln Gln Gln Ile Val Asp
        210                 215                 220
His Ile Gln Pro Val Lys Gln Glu Phe Ile Pro Ala Glu Arg Asp Glu
225                 230                 235                 240
Ala Leu Val Glu Arg Ile Lys Ser Leu Thr Glu Glu Lys Gly Leu Lys
                245                 250                 255
Glu Thr Val Leu Thr Phe Asp Lys Gln Gln Arg Asp Glu Asn Leu Asp
                260                 265                 270
Asn Leu Lys Glu Glu Ile Val Asn Glu Phe Ile Asp Glu Glu Asp Pro
        275                 280                 285
Glu Asn Glu Leu Leu Ile Lys Glu Val Tyr Ala Ile Leu Asn Glu Leu
        290                 295                 300
Val Lys Glu Glu Val Arg Arg Leu Ile Ala Asp Glu Lys Ile Arg Pro
```

```
         305                    310                    315                    320
Asp Gly Arg Lys Pro Asp Glu Ile Arg Pro Leu Asp Ser Glu Val Gly
                    325                    330                    335
Ile Leu Pro Arg Thr His Gly Ser Gly Leu Phe Thr Arg Gly Gln Thr
            340                    345                    350
Gln Ala Leu Ser Val Leu Thr Leu Gly Ala Leu Gly Asp Tyr Gln Leu
        355                    360                    365
Ile Asp Gly Leu Gly Pro Glu Glu Glu Lys Arg Phe Met His His Tyr
    370                    375                    380
Asn Phe Pro Asn Phe Ser Val Gly Glu Thr Gly Pro Val Arg Ala Pro
385                    390                    395                    400
Gly Arg Arg Glu Ile Gly His Gly Ala Leu Gly Glu Arg Ala Leu Lys
                405                    410                    415
Tyr Ile Ile Pro Asp Thr Ala Asp Phe Pro Tyr Thr Ile Arg Ile Val
            420                    425                    430
Ser Glu Val Leu Glu Ser Asn Gly Ser Ser Ser Gln Ala Ser Ile Cys
        435                    440                    445
Gly Ser Thr Leu Ala Leu Met Asp Ala Gly Val Pro Ile Lys Ala Pro
    450                    455                    460
Val Ala Gly Ile Ala Met Gly Leu Val Thr Arg Glu Asp Ser Tyr Thr
465                    470                    475                    480
Ile Leu Thr Asp Ile Gln Gly Met Glu Asp Ala Leu Gly Asp Met Asp
                485                    490                    495
Phe Lys Val Ala Gly Thr Lys Glu Gly Ile Thr Ala Ile Gln Met Asp
                500                    505                    510
Ile Lys Ile Asp Gly Leu Thr Arg Glu Ile Ile Glu Glu Ala Leu Glu
        515                    520                    525
Gln Ala Arg Arg Gly Arg Leu Glu Ile Met Asn His Met Leu Gln Thr
        530                    535                    540
Ile Asp Gln Pro Arg Thr Glu Leu Ser Ala Tyr Ala Pro Lys Val Val
545                    550                    555                    560
Thr Met Thr Ile Lys Pro Asp Lys Ile Arg Asp Val Ile Gly Pro Gly
                565                    570                    575
Gly Lys Lys Ile Asn Glu Ile Ile Asp Glu Thr Gly Val Lys Leu Asp
            580                    585                    590
Ile Glu Gln Asp Gly Thr Ile Phe Ile Gly Ala Val Asp Gln Ala Met
        595                    600                    605
Ile Asn Arg Ala Arg Glu Ile Ile Glu Glu Ile Thr Arg Glu Ala Glu
    610                    615                    620
Val Gly Gln Thr Tyr Gln Ala Thr Val Lys Arg Ile Glu Lys Tyr Gly
625                    630                    635                    640
```

Ala Phe Val Gly Leu Phe Pro Gly Lys Asp Ala Leu Leu His Ile Ser
                  645               650                    655

Gln Ile Ser Lys Asn Arg Ile Glu Lys Val Glu Asp Val Leu Lys Ile
                  660               665                    670

Gly Asp Thr Ile Glu Val Lys Ile Thr Glu Ile Asp Lys Gln Gly Arg
                  675               680                    685

Val Asn Ala Ser His Arg Ala Leu Glu Glu
              690               695


    <210> 47

    <211> 578

    <212> PRT

    <213> Staphylococcus aureus


    <400> 47

Met Ile Lys Arg Tyr Leu Gln Phe Val Lys Pro Tyr Lys Tyr Arg Ile
  1                 5                 10                    15

Phe Ala Thr Ile Ile Val Gly Ile Ile Lys Phe Gly Ile Pro Met Leu
                  20                25                    30

Ile Pro Leu Leu Ile Lys Tyr Ala Ile Asp Gly Val Ile Asn Asn His
          35                40                    45

Ala Leu Thr Thr Asp Glu Lys Val His His Leu Thr Ile Ala Ile Gly
      50                55                    60

Ile Ala Leu Phe Ile Phe Val Ile Val Arg Pro Pro Ile Glu Phe Ile
65                70                75                    80

Arg Gln Tyr Leu Ala Gln Trp Thr Ser Asn Lys Ile Leu Tyr Asp Ile
                  85                90                    95

Arg Lys Lys Leu Tyr Asn His Leu Gln Ala Leu Ser Ala Arg Phe Tyr
                  100               105                   110

Ala Asn Asn Gln Val Gly Gln Val Ile Ser Arg Val Ile Asn Asp Val
          115               120               125

Glu Gln Thr Lys Asp Phe Ile Leu Thr Gly Leu Met Asn Ile Trp Leu
          130               135               140

Asp Cys Ile Thr Ile Ile Ile Ala Leu Ser Ile Met Phe Phe Leu Asp
145               150               155                   160

Val Lys Leu Thr Leu Ala Ala Leu Phe Ile Phe Pro Phe Tyr Ile Leu
                  165               170                   175

Thr Val Tyr Val Phe Phe Gly Arg Leu Arg Lys Leu Thr Arg Glu Arg
                  180               185                   190

Ser Gln Ala Leu Ala Glu Val Gln Gly Phe Leu His Glu Arg Val Gln
          195               200               205

```
Gly Ile Ser Val Val Lys Ser Phe Ala Ile Glu Asp Asn Glu Ala Lys
    210                 215                 220
Asn Phe Asp Lys Lys Asn Thr Asn Phe Leu Thr Arg Ala Leu Lys His
225                 230                 235                 240
Thr Arg Trp Asn Ala Tyr Ser Phe Ala Ala Ile Asn Thr Val Thr Asp
                245                 250                 255
Ile Gly Pro Ile Ile Val Ile Gly Val Gly Ala Tyr Leu Ala Ile Ser
                260                 265                 270
Gly Ser Ile Thr Val Gly Thr Leu Ala Ala Phe Val Gly Tyr Leu Glu
            275                 280                 285
Leu Leu Phe Gly Pro Leu Arg Arg Leu Val Ala Ser Phe Thr Thr Leu
            290                 295                 300
Thr Gln Ser Phe Ala Ser Met Asp Arg Val Phe Gln Leu Ile Asp Glu
305                 310                 315                 320
Asp Tyr Asp Ile Lys Asn Gly Val Gly Ala Gln Pro Ile Glu Ile Lys
                325                 330                 335
Gln Gly Arg Ile Asp Ile Asp His Val Ser Phe Gln Tyr Asn Asp Asn
            340                 345                 350
Glu Ala Pro Ile Leu Lys Asp Ile Asn Leu Ser Ile Glu Lys Gly Glu
            355                 360                 365
Thr Val Ala Phe Val Gly Met Ser Gly Gly Gly Lys Ser Thr Leu Ile
    370                 375                 380
Asn Leu Ile Pro Arg Phe Tyr Asp Val Thr Ser Gly Gln Ile Leu Ile
385                 390                 395                 400
Asp Gly His Asn Ile Lys Asp Phe Leu Thr Gly Ser Leu Arg Asn Gln
                405                 410                 415
Ile Gly Leu Val Gln Gln Asp Asn Ile Leu Phe Ser Asp Thr Val Lys
                420                 425                 430
Glu Asn Ile Leu Leu Gly Arg Pro Thr Ala Thr Asp Glu Glu Val Val
            435                 440                 445
Glu Ala Ala Lys Met Ala Asn Ala His Asp Phe Ile Met Thr Leu Pro
            450                 455                 460
Gln Gly Tyr Asp Thr Glu Val Gly Glu Arg Gly Val Lys Leu Ser Gly
465                 470                 475                 480
Gly Gln Lys Gln Arg Leu Ser Ile Ala Arg Ile Phe Leu Asn Asn Pro
                485                 490                 495
Pro Ile Leu Ile Leu Asp Glu Ala Thr Ser Ala Leu Asp Leu Glu Ser
                500                 505                 510
Glu Ser Ile Ile Gln Glu Ala Leu Asp Val Leu Ser Lys Asp Arg Thr
            515                 520                 525
Thr Leu Ile Val Ala His Arg Leu Ser Thr Ile Thr His Ala Asp Lys
```

```
                530                      535                     540
Ile Val Val Ile Glu Asn Gly His Ile Val Glu Thr Gly Thr His Arg
545                      550                     555                     560
Glu Leu Ile Ala Lys Gln Gly Ala Tyr Glu His Leu Tyr Ser Ile Gln
                565                     570                     575
Asn Leu


        <210> 48
        <211> 144
        <212> PRT
        <213> Staphylococcus aureus


        <400> 48
Met Asp Arg Thr Lys Gln Ser Leu Asn Val Phe Val Gly Met Asn Arg
 1               5                   10                  15
Ala Leu Asp Thr Leu Glu Gln Ile Thr Lys Glu Asp Val Lys Arg Tyr
            20                  25                  30
Gly Leu Asn Ile Thr Glu Phe Ala Val Leu Glu Leu Leu Tyr Asn Lys
        35                  40                  45
Gly Pro Gln Pro Ile Gln Arg Ile Arg Asp Arg Val Leu Ile Ala Ser
        50                  55                  60
Ser Ser Ile Ser Tyr Val Val Ser Gln Leu Glu Asp Lys Gly Trp Ile
65                  70                  75                  80
Thr Arg Glu Lys Asp Lys Asp Asp Lys Arg Val Tyr Met Ala Cys Leu
            85                  90                  95
Thr Glu Lys Gly Gln Ser Gln Met Ala Asp Ile Phe Pro Lys His Ala
            100                 105                 110
Glu Thr Leu Thr Lys Ala Phe Asp Val Leu Thr Lys Asp Glu Leu Thr
        115                 120                 125
Ile Leu Gln Gln Ala Phe Lys Lys Leu Ser Ala Gln Ser Thr Glu Val
        130                 135                 140
```

## Description

### RELATED APPLICATIONS

This application claims benefit to US Provisional Patent Application Number 60/055,387, filed August 5, 1997.

### FIELD OF THE INVENTION

This invention relates to newly identified polynucleotides and polypeptides, and their production and uses, as well as their variants, agonists and antagonists, and their uses. In particular, in these and in other regards, the invention relates to novel polynucleotides and polypeptides set forth in the Sequence Listing.

### BACKGROUND OF THE INVENTION

It is particularly preferred to employ Staphylococcal genes and gene products as targets for the development of antibiotics. The Staphylococci make up a medically important genera of microbes. They are known to produce two types of disease, invasive and toxigenic. Invasive infections are characterized generally by abscess formation effecting both skin surfaces and deep tissues. *Staphylococcus aureus* (*S. aureus*) is the second leading cause of bacteremia in cancer patients. Osteomyelitis, septic arthritis, septic thrombophlebitis and acute bacterial endocarditis are also relatively common. There are at least three clinical conditions resulting from the toxigenic properties of Staphylococci. The manifestation of these diseases result from the actions of exotoxins as opposed to tissue invasion and bacteremia. These conditions include: Staphylococcal food poisoning, scalded skin syndrome and toxic shock syndrome.

The frequency of *Staphylococcus aureus* infections has risen dramatically in the past 20 years. This has been attributed to the emergence of multiply antibiotic resistant strains and an increasing population of people with weakened immune systems. It is no longer uncommon to isolate *Staphylococcus aureus* strains which are resistant to some or all of the standard antibiotics. This has created a demand for both new anti-microbial agents and diagnostic tests for this organism.

Substantial effort has been invested this century in the successful discovery and development of antibacterials. Paradoxically, although antibacterials are devised to eradicate infection in mammals we know almost nothing of the physiology of bacterial pathogens in infective situations in the host. Using sequences from the *Staphylococcus aureus* chromosome we have developed an RT-PCR based procedure which allows us to identify those bacterial genes transcribed at any stage of infection and also from different niches of infection. The derivation of such information is a critical step in understanding the global response of the bacterial gene complement to the host environment. From the knowledge of bacterial genes both of known and unknown function which are widely transcribed in the host it is possible to attempt to ascertain by database searching those which are present only in the eubacteria. Further prioritization of such genes by consideration of the likely role of their products towards the maintenance of infection and the facility of setting up a screen for inhibitors of the biochemical function indicated by their homology to characterized genes allows the compilation of a list for gene essentiality studies using genetic deletion or controlled regulation techniques. The proteins expressed by genes shown to be necessary for growth *in vitro* or in pathogenesis in animal models provide novel targets for antibacterial screening to find agents which are broadly inhibitory towards pathogenesis. This invention provides *S.aureus* WCUH 29 polynucleotides which are transcribed in infected tissue, in particular in both acute and chronic infections.

Clearly, there is a need for factors, such as the novel compounds of the invention, that have a present benefit of being useful to screen compounds for antibiotic activity. Such factors are also useful to determine their role in pathogenesis of infection, dysfunction and disease. There is also a need for identification and characterization of such factors and their antagonists and agonists which can play a role in preventing, ameliorating or correcting infections, dysfunctions or diseases.

The polypeptides of the invention have amino acid sequence homology to a known protein(s) as set forth in Table 1.

### SUMMARY OF THE INVENTION

It is an object of the invention to provide polypeptides that have been identified as novel polypeptides by homology between an amino acid sequence selected from the group consisting of the sequences set out in the Sequence Listing and a known amino acid sequence or sequences of other proteins such as the protein identities listed in Table 1.

It is a further object of the invention to provide polynucleotides that encode novel polypeptides, particularly polynucleotides that encode polypeptides of *Staphylococcus aureus.*

In a particularly preferred embodiment of the invention, the polynucleotide comprises a region encoding a polypeptide comprising a sequence selected from the group consisting of the sequences set out in the Sequence Listing, or a variant of any of these sequences.

In another particularly preferred embodiment of the invention, there is a novel protein from *Staphylococcus aureus* comprising an amino acid sequence selected from the group consisting of the sequences set out in the Sequence Listing, or a variant of any of these sequences.

In accordance with another aspect of the invention, there is provided an isolated nucleic acid molecule encoding a mature polypeptide expressible by the *Staphylococcus aureus* WCUH 29 strain contained in the deposited strain.

As a further aspect of the invention, there are provided isolated nucleic acid molecules encoding a polypeptide of the invention, particularly *Staphylococcus aureus* polypeptide, and including mRNAs, cDNAs, genomic DNAs. Further embodiments of the invention include biologically, diagnostically, prophylactically, clinically or therapeutically useful variants thereof, and compositions comprising the same.

In accordance with another aspect of the invention, there is provided the use of a polynucleotide of the invention for therapeutic or prophylactic purposes, in particular genetic immunization. Among the particularly preferred embodiments of the invention are naturally occurring allelic variants of a polypeptide of the invention and polypeptides encoded thereby.

As another aspect of the invention, there are provided novel polypeptides of *Staphylococcus aureus* as well as biologically, diagnostically, prophylactically, clinically or therapeutically useful variants thereof, and compositions comprising the same.

Among the particularly preferred embodiments of the invention are variants of the polypeptides of the invention encoded by naturally occurring alleles of their genes.

In a preferred embodiment of the invention, there are provided methods for producing the aforementioned polypeptides.

In accordance with yet another aspect of the invention, there are provided inhibitors to such polypeptides, useful as antibacterial agents, including, for example, antibodies.

In accordance with certain preferred embodiments of the invention, there are provided products, compositions and methods for assessing expression of the polypeptides and polynucleotides of the invention, treating disease, for example, disease, such as, infections of the upper respiratory tract (e.g., otitis media, bacterial tracheitis, acute epiglottitis, thyroiditis), lower respiratory (e.g., empyema, lung abscess), cardiac (e.g., infective endocarditis), gastrointestinal (e.g., secretory diarrhoea, splenic absces, retroperitoneal abscess), CNS (e.g., cerebral abscess), eye (e.g., blepharitis, conjunctivitis, keratitis, endophthalmitis, preseptal and orbital cellulitis, darcryocystitis), kidney and urinary tract (e.g., epididymitis, intrarenal and perinephric abscess, toxic shock syndrome), skin (e.g., impetigo, folliculitis, cutaneous abscesses, cellulitis, wound infection, bacterial myositis) bone and joint (e.g., septic arthritis, osteomyelitis), assaying genetic variation, and administering a polypeptide or polynucleotide of the invention to an organism to raise an immunological response against a bacteria, especially a *Staphylococcus aureus* bacteria.

In accordance with certain preferred embodiments of this and other aspects of the invention, there are provided polynucleotides that hybridize to a polynucleotide sequence of the invention, particularly under stringent conditions.

In certain preferred embodiments of the invention, there are provided antibodies against polypeptides of the invention.

In other embodiments of the invention, there are provided methods for identifying compounds which bind to or otherwise interact with and inhibit or activate an activity of a polypeptide or polynucleotide of the invention comprising: contacting a polypeptide or polynucleotide of the invention with a compound to be screened under conditions to permit binding to or other interaction between the compound and the polypeptide or polynucleotide to assess the binding to or other interaction with the compound, such binding or interaction being associated with a second component capable of providing a detectable signal in response to the binding or interaction of the polypeptide or polynucleotide with the compound; and determining whether the compound binds to or otherwise interacts with and activates or inhibits an activity of the polypeptide or polynucleotide by detecting the presence or absence of a signal generated from the binding or interaction of the compound with the polypeptide or polynucleotide.

In accordance with yet another aspect of the invention, there are provided agonists and antagonists of the polypeptides and polynucleotides of the invention, preferably bacteriostatic or bacteriocidal agonists and antagonists.

In a further aspect of the invention, there are provided compositions comprising a polynucleotide or a polypeptide of the invention for administration to a cell or to a multicellular organism.

Various changes and modifications within the spirit and scope of the disclosed invention will become readily apparent to those skilled in the art from reading the following descriptions and from reading the other parts of the present disclosure.

**GLOSSARY**

The following definitions are provided to facilitate understanding of certain terms used frequently herein.

"Host cell" is a cell which has been transformed or transfected, or is capable of transformation or transfection by an exogenous polynucleotide sequence.

"Identity," as known in the art, is a relationship between two or more polypeptide sequences or two or more polynucleotide sequences, as the case may be, as determined by comparing the sequences. In the art, "identity" also means the degree of sequence relatedness between polypeptide or polynucleotide sequences, as the case may be, as determined by the match between strings of such sequences. "Identity" can be readily calculated by known methods, including but not limited to those described in (*Computational Molecular Biology,* Lesk, A.M., ed., Oxford University Press, New York, 1988; *Biocomputing: Informatics and Genome Projects,* Smith, D.W., ed., Academic Press, New York, 1993; *Computer Analysis ofSequence Data,* Part I, Griffin, A.M., and Griffin, H.G., eds., Humana Press, New Jersey, 1994; *Sequence Analysis in Molecular Biology,* von Heinje, G., Academic Press, 1987; and *Sequence Analysis Primer,* Gribskov, M. and Devereux, J., eds., M Stockton Press, New York, 1991; and Carillo, H., and Lipman, D., SIAM *J. Applied Math., 48:* 1073 (1988). Methods to determine identity are designed to give the largest match between the sequences tested. Moreover, methods to determine identity are codified in publicly available computer programs. Computer program methods to determine identity between two sequences include, but are not limited to, the GCG program package (Devereux, J., et al., *Nucleic Acids Research 12(1):* 387 (1984)), BLASTP, BLASTN, and FASTA (Altschul, S.F. et al., *J. Molec. Biol. 215:* 403-410 (1990). The BLAST X program is publicly available from NCBI and other sources *(BLAST Manual,* Altschul, S., *et al.,* NCBI NLM NIH Bethesda, MD 20894; Altschul, S., *et al., J. Mol. Biol. 215:* 403-410 (1990). The well known Smith Waterman algorithm may also be used to determine identity.

Parameters for polypeptide sequence comparison include the following: Algorithm: Needleman and Wunsch, J. Mol Biol. 48: 443-453 (1970)

Comparison matrix: BLOSSUM62 from Hentikoff and Hentikoff, Proc. Natl. Acad. Sci. USA. 89:10915-10919 (1992)
Gap Penalty: 12
Gap Length Penalty: 4

A program useful with these parameters is publicly available as the "gap" program from Genetics Computer Group, Madison WI. The aforementioned parameters are the default parameters for peptide comparisons (along with no penalty for end gaps).

Parameters for polynucleotide comparison include the following: Algorithm: Needleman and Wunsch, J. Mol Biol. 48: 443-453 (1970)

Comparison matrix: matches = +10, mismatch = 0
Gap Penalty: 50
Gap Length Penalty: 3
Available as: The "gap" program from Genetics Computer Group, Madison WI. These are the default parameters for nucleic acid comparisons.

A preferred meaning for "identity" for polynucleotides and polypeptides, as the case may be, are provided in (1) and (2) below.

(1) Polynucleotide embodiments further include an isolated polynucleotide comprising a polynucleotide sequence having at least a 50, 60, 70, 80, 85, 90, 95, 97 or 100% identity to the reference sequence of SEQ ID NO: 1, wherein said polynucleotide sequence may be identical to the reference sequence of SEQ ID NO: 1 or may include up to a certain integer number of nucleotide alterations as compared to the reference sequence, wherein said alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence, and wherein said number of nucleotide alterations is determined by multiplying the total number of nucleotides in SEQ ID NO: 1 by the integer defining the percent identity divided by 100 and then subtracting that product from said total number of nucleotides in SEQ ID NO: 1, or:

$$n_n \leq x_n - (x_n \bullet y),$$

wherein $n_n$ is the number of nucleotide alterations, $x_n$ is the total number of nucleotides in SEQ ID NO:1, $y$ is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and • is the symbol for the multiplication operator, and wherein any non-integer product of $x_n$ and $y$ is rounded down to the nearest integer prior to subtracting it from $x_n$. Alterations of a polynucleotide sequence encoding the polypeptide of SEQ ID NO:2 may create nonsense, missense or frameshift mutations in this coding sequence and thereby alter the polypeptide encoded by the polynucleotide following such alterations. By way of example, a polynucleotide sequence of the present invention may be identical to the reference sequence of SEQ ID NO: 1, that is it may be 100% identical, or it may include up to a certain integer number of nucleic acid alterations as compared to the reference sequence such that the percent identity is less than 100% identity. Such alterations are selected from the group consisting of at least one nucleic acid deletion, substitution, including transition and transversion, or insertion, and wherein said alterations may occur at the 5' or 3' terminal positions of the reference polynucleotide sequence or anywhere between those terminal positions, interspersed either individually among the nucleic acids in the reference sequence or in one or more contiguous groups within the reference sequence. The number of nucleic acid alterations for a given percent identity is determined by multiplying the total number of nucleic acids in SEQ ID NO: 1 by the integer defining the percent identity divided by 100 and then subtracting that product from said total number of nucleic acids in SEQ ID NO: 1, or:

$$n_n \leq x_n - (x_n \bullet y),$$

wherein $n_n$ is the number of nucleic acid alterations, $x_n$ is the total number of nucleic acids in SEQ ID NO: I, $y$ is, for instance 0.70 for 70%, 0.80 for 80%, 0.85 for 85% etc., • is the symbol for the multiplication operator, and wherein any non-integer product of $x_n$ and $y$ is rounded down to the nearest integer prior to subtracting it from $x_n$.

(2) Polypeptide embodiments further include an isolated polypeptide comprising a polypeptide having at least a 50,60, 70, 80, 85, 90, 95, 97 or 100% identity to a polypeptide reference sequence of SEQ ID NO:2, wherein said polypeptide sequence may be identical to the reference sequence of SEQ ID NO:2 or may include up to a certain integer number of amino acid alterations as compared to the reference sequence, wherein said alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence, and wherein said number of amino acid alterations is determined by multiplying the total number of amino acids in SEQ ID NO:2 by the integer defining the percent identity divided by 100 and then subtracting that product from said total number of amino acids in SEQ ID NO:2, or:

$$n_a \leq x_a - (x_a \bullet y),$$

wherein $n_a$ is the number of amino acid alterations, $x_a$ is the total number of amino acids in SEQ ID NO:2, $y$ is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and • is the symbol for the multiplication operator, and wherein any non-integer product of $x_a$ and $y$ is rounded down to the nearest integer prior to subtracting it from $x_a$.

By way of example, a polypeptide sequence of the present invention may be identical to the reference sequence of SEQ ID NO:2, that is it may be 100% identical, or it may include up to a certain integer number of amino acid alterations as compared to the reference sequence such that the percent identity is less than 100% identity. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in SEQ ID NO:2 by the integer defining the percent identity divided by 100 and then subtracting that product from said total number of amino acids in SEQ ID NO:2, or:

$$n_a \leq x_a - (x_a \bullet y),$$

wherein $n_a$ is the number of amino acid alterations, $x_a$ is the total number of amino acids in SEQ ID NO:2, $y$ is, for instance 0.70 for 70%, 0.80 for 80%, 0.85 for 85% etc., and • is the symbol for the multiplication operator, and wherein any non-integer product of $x_a$ and $y$ is rounded down to the nearest integer prior to subtracting it from $x_a$.

"Isolated" means altered "by the hand of man" from its natural state, *i.e.,* if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein.

"Polynucleotide(s)" generally refers to any polyribonucleotide or polydeoxribonucleotide, which may be unmodified RNA or DNA or modified RNA or DNA. "Polynucleotide(s)" include, without limitation, single- and double-stranded DNA, DNA that is a mixture of single- and double-stranded regions or single-, double- and triple-stranded regions, single- and double-stranded RNA, and RNA that is mixture of single- and double-stranded regions, hybrid molecules comprising DNA and RNA that may be single-stranded or, more typically, double-stranded, or triple-stranded regions, or a mixture of single- and double-stranded regions. In addition, "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. As used herein, the term "polynucleotide(s)" also includes DNAs or RNAs as described above that contain one or more modified bases. Thus, DNAs or RNAs with backbones modified for stability or for other reasons are "polynucleotide(s)" as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term "polynucleotide(s)" as it is employed herein embraces such chemically, enzymatically or metabolically modified forms of polynucleotides, as well as the chemical forms of DNA and RNA characteristic of viruses and cells, including, for example, simple and complex cells. "Polynucleotide(s) " also embraces short polynucleotides often referred to as oligonucleotide(s).

"Polypeptide(s)" refers to any peptide or protein comprising two or more amino acids joined to each other by peptide bonds or modified peptide bonds. "Polypeptide(s)" refers to both short chains, commonly referred to as peptides, oligopeptides and oligomers and to longer chains generally referred to as proteins. Polypeptides may contain amino acids other than the 20 gene encoded amino acids. "Polypeptide(s)" include those modified either by natural processes, such as processing and other post-translational modifications, but also by chemical modification techniques. Such modifications are well described in basic texts and in more detailed monographs, as well as in a voluminous research literature, and they are well known to those of skill in the art. It will be appreciated that the same type of modification may be present in the same or varying degree at several sites in a given polypeptide. Also, a given polypeptide may contain many types of modifications. Modifications can occur anywhere in a polypeptide, including the peptide backbone, the amino acid side-chains, and the amino or carboxyl termini. Modifications include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, glycosylation, lipid attachment, sulfation, gamma-carboxylation of glutamic acid residues, hydroxylation and ADP-ribosylation, selenoylation, sulfation, transfer-RNA mediated addition of amino acids to proteins, such as arginylation, and ubiquitination. See, for instance, *PROTEINS - STRUCTURE AND MOLECULAR PROPERTIES,* 2nd Ed., T. E. Creighton, W. H. Freeman and Company, New York (1993) and Wold, F., Posttranslational Protein Modifications: Perspectives and Prospects, pgs. 1-12 in *POSTTRANSLATIONAL COVALENT MODIFICATION OF PROTEINS,* B. C. Johnson, Ed., Academic Press, New York (1983); Seifter et al., *Meth. Enzymol. 182:*626-646 (1990) and Rattan et al., *Protein Synthesis: Posttranslational Modifications and Aging,* Ann. N.Y. Acad. Sci. 663: 48-62 (1992). Polypeptides may be branched or cyclic, with or without branching. Cyclic, branched and branched circular polypeptides may result from post-translational natural processes and may be made by entirely synthetic methods, as well.

"Variant(s)" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques, by direct synthesis, and by other recombinant methods known to skilled artisans.

## DESCRIPTION OF THE INVENTION

Each of polynucleotide and polypeptide sequences provided herein may be used in the discovery and development of antibacterial compounds. Upon expression of the sequences with the appropriate initiation and termination codons the encoded polypeptide can be used as a target for the screening of antimicrobial drugs. Additionally, the DNA sequences encoding preferably the amino terminal regions of the encoded protein or the Shine-Delgarno region can be used to construct antisense sequences to control the expression of the coding sequence of interest. Furthermore, many of the sequences disclosed herein also provide regions upstream and downstream from the encoding sequence. These sequences are useful as a source of regulatory elements for the control of bacterial gene expression. Such sequences are conveniently isolated by restriction enzyme action or synthesized chemically and introduced, for example, into promoter identification strains. These strains contain a reporter structural gene sequence located downstream from a restriction site such that if an active promoter is inserted, the reporter gene will be expressed.

Although each of the sequences may be employed as described above, this invention also provides several means for identifying particularly useful target genes. The first of these approaches entails searching appropriate databases for sequence matches in related organisms. Thus, if a homologue exists, the Staphyococcal-like form of this gene would likely play an analogous role. For example, a Staphyococcal protein identified as homologous to a cell surface protein in another organism would be useful as a vaccine candidate. To the extent such homologies have been identified for the sequences disclosed herein they are reported along with the encoding sequence.

## ORF Gene Expression

Recently techniques have become available to evaluate temporal gene expression in bacteria, particularly as it applies to viability under laboratory and infection conditions. A number of methods can be used to identify genes which are essential to survival *per se,* or essential to the establishment/maintenance of an infection. Identification of an ORF unknown by one of these methods yields additional information about its function and permits the selection of such an ORF for further development as a screening target. Briefly, these approaches include:

1) **Signature Tagged Mutagenesis (STM):** This technique is described by Hensel et al., Science 269: 400-403 (1995), the contents of which is incorporated by reference for background purposes. Signature tagged mutagenesis identifies genes necessary for the establishment/maintenance of infection in a given infection model.

The basis of the technique is the random mutagenesis of target organism by various means (e.g., transposons) such that unique DNA sequence tags are inserted in close proximity to the site of mutation. The tags from a mixed population of bacterial mutants and bacteria recovered from an infected hosts are detected by amplification, radiolabeling and hybridisation analysis. Mutants attenuated in virulence are revealed by absence of the tag from the pool of bacteria recovered from infected hosts.

In *Staphylococcus aureus,* because the transposon system is less well developed, a more efficient way of creating the tagged mutants is to use the insertion-duplication mutagenesis technique as described by Morrison et al., J. Bacteriol. 159:870 (1984) the contents of which is incorporated by reference for background purposes.

2) **In Vivo Expression Technology (IVET)**: This technique is described by Camilli et al., Proc. Nat'l. Acad. Sci. USA. 91:2634-2638 (1994), the contents of which is incorporated by reference for background purposes. IVET identifies genes up-regulated during infection when compared to laboratory cultivation, implying an important role in infection. ORF identified by this technique are implied to have a significant role in infection establishment/maintenance.

In this technique random chromosomal fragments of target organism are cloned upstream of a promoter-less recombinase gene in a plasmid vector. This construct is introduced into the target organism which carries an antibiotic resistance gene flanked by resolvase sites. Growth in the presence of the antibiotic removes from the population those fragments cloned into the plasmid vector capable of supporting transcription of the recombinase gene and therefore have caused loss of antibiotic resistance. The resistant pool is introduced into a host and at various times after infection bacteria may be recovered and assessed for the presence of antibiotic resistance. The chromosomal fragment carried by each antibiotic sensitive bacterium should carry a promoter or portion of a gene normally upregulated during infection. Sequencing upstream of the recombinase gene allows identification of the up regulated gene.

3) **Differential display:** This technique is described by Chuang et al., J. Bacteriol. 175:2026-2036 (1993), the contents of which is incorporated by reference for background purposes. This method identifies those genes which are expressed in an organism by identifying mRNA present using randomly-primed RT-PCR. By comparing pre-infection and post infection profiles, genes up and down regulated during infection can be identified and the RT-PCR product sequenced and matched to ORF 'unknowns'.

**4) Generation of conditional lethal mutants by transposon mutagenesis**: This technique, described by de Lorenzo, V. et al., Gene 123:17-24 (1993); Neuwald, A. F. et al., Gene 125: 69-73 (1993); and Takiff, H. E. et al., J. Bacteriol. 174:1544-1553 (1992), the contents of which is incorporated by reference for background purposes, identifies genes whose expression are essential for cell viability.

In this technique transposons carrying controllable promoters, which provide transcription outward from the transposon in one or both directions, are generated. Random insertion of these transposons into target organisms and subsequent isolation of insertion mutants in the presence of inducer of promoter activity ensures that insertions which separate promoter from coding region of a gene whose expression is essential for cell viability will be recovered. Subsequent replica plating in the absence of inducer identifies such insertions, since they fail to survive. Sequencing of the flanking regions of the transposon allows identification of site of insertion and identification of the gene disrupted. Close monitoring of the changes in cellular processes/morphology during growth in the absence of inducer yields information on likely function of the gene. Such monitoring could include flow cytometry (cell division, lysis, redox potential, DNA replication), incorporation of radiochemically labeled precursors into DNA, RNA, protein, lipid, peptidoglycan, monitoring reporter enzyme gene fusions which respond to known cellular stresses.

**5) Generation of conditional lethal mutants by chemical mutagenesis**: This technique is described by Beckwith, J., Methods in Enzymology 204: 3-18 (1991), the contents of which are incorporated herein by reference for background purposes. In this technique random chemical mutagenesis of target organism, growth at temperature other than physiological temperature (permissive temperature) and subsequent replica plating and growth at different temperature (e.g. 42°C to identify ts, 25°C to identify cs) are used to identify those isolates which now fail to grow (conditional mutants). As above close monitoring of the changes upon growth at the non-permissive temperature yields information on the function of the mutated gene. Complementation of conditional lethal mutation by library from target organism and sequencing of complementing gene allows matching with unknown ORF.

**6) RT-PCR**: *Staphylococcus aureus* messenger RNA is isolated from bacterial infected tissue e.g. 48 hour murine lung infections, and the amount of each mRNA species assessed by reverse transcription of the RNA sample primed with random hexanucleotides followed by PCR with gene specific primer pairs. The determination of the presence and amount of a particular mRNA species by quantification of the resultant PCR product provides information on the bacterial genes which are transcribed in the infected tissue. Analysis of gene transcription can be carried out at different times of infection to gain a detailed knowledge of gene regulation in bacterial pathogenesis allowing for a clearer understanding of which gene products represent targets for screens for novel antibacterials. Because of the gene specific nature of the PCR primers employed it should be understood that the bacterial mRNA preparation need not be free of mammalian RNA. This allows the investigator to carry out a simple and quick RNA preparation from infected tissue to obtain bacterial mRNA species which are very short lived in the bacterium (in the order of 2 minute halflives). Optimally the bacterial mRNA is prepared from infected murine lung tissue by mechanical disruption in the presence of TRIzole (GIBCO-BRL) for very short periods of time, subsequent processing according to the manufacturers of TRIzole reagent and DNAase treatment to remove contaminating DNA. Preferably the process is optimised by finding those conditions which give a maximum amount of *Staphylococcus aureus* 16S ribosomal RNA as detected by probing Northerns with a suitably labelled sequence specific oligonucleotide probe. Typically a 5' dye labelled primer is used in each PCR primer pair in a PCR reaction which is terminated optimally between 8 and 25 cycles. The PCR products are separated on 6% polyacrylamide gels with detection and quantification using GeneScanner (manufactured by ABI).

Each of these techniques may have advantages or disadvantage depending on the particular application. The skilled artisan would choose the approach that is the most relevant with the particular end use in mind. For example, some genes might be recognized as essential for infection but in reality are only necessary for the initiation of infection and so their products would represent relatively unattractive targets for antibacterials developed to cure established and chronic infections.

Use of the of these technologies when applied to the ORFs of the present invention enables identification of bacterial proteins expressed during infection, inhibitors of which would have utility in anti-bacterial therapy.

The invention relates to novel polypeptides and polynucleotides as described in greater detail below. In particular, the invention relates to polypeptides and polynucleotides of *Staphylococcus aureus,* which is related by amino acid sequence homology to known polypeptide as set forth in Table 1. The invention relates especially to compounds having the nucleotide and amino acid sequence selected from the group consisting of the sequences set out in the Sequence Listing, and to the nucleotide sequences of the DNA in the deposited strain and amino acid sequences encoded thereby.

## Deposited materials

A deposit containing a *Staphylococcus aureus* WCUH 29 strain has been deposited with the National Collections of Industrial and Marine Bacteria Ltd. (herein "NCIMB"), 23 St. Machar Drive, Aberdeen AB2 1RY, Scotland on 11 September 1995 and assigned NCIMB Deposit No. 40771, and referred to as *Staphylococcus aureus* WCUH29 on deposit. The *Staphylococcus aureus* strain deposit is referred to herein as "the deposited strain" or as "the DNA of the deposited strain."

The deposited strain contains the full length genes comprising the polynucleotides set forth in the Sequence Listing. The sequence of the polynucleotides contained in the deposited strain, as well as the amino acid sequence of the polypeptide encoded thereby, are controlling in the event of any conflict with any description of sequences herein.

The deposit of the deposited strain has been made under the terms of the Budapest Treaty on the International Recognition of the Deposit of Micro-organisms for Purposes of Patent Procedure. The strain will be irrevocably and without restriction or condition released to the public upon the issuance of a patent. The deposited strain is provided merely as convenience to those of skill in the art and is not an admission that a deposit is required for enablement, such as that required under 35 U.S.C. §112.

A license may be required to make, use or sell the deposited strain, and compounds derived therefrom, and no such license is hereby granted.

## Polypeptides

The polypeptides of the invention include the polypeptides set forth in the Sequence Listing (in particular the mature polypeptide) as well as polypeptides and fragments, particularly those which have the biological activity of a polypeptide of the invention, and also those which have at least 50%, 60% or 70% identity to a polypeptide sequence selected from the group consisting of the sequences set out in the Sequence Listing or the relevant portion, preferably at least 80% identity to a polypeptide sequence selected from the group consisting of the sequences set out in the Sequence Listing, and more preferably at least 90% similarity (more preferably at least 90% identity) to a polypeptide sequence selected from the group consisting of the sequences set out in the Sequence Listing, and still more preferably at least 95% similarity (still more preferably at least 95% identity) to a polypeptide sequence selected from the group consisting of the sequences set out in the Sequence Listing, and also include portions of such polypeptides with such portion of the polypeptide generally containing at least 30 amino acids and more preferably at least 50 amino acids.

The invention also includes polypeptides of the formula:

$$X\text{-}(R_1)_n\text{-}(R_2)\text{-}(R_3)_n\text{-}Y$$

wherein, at the amino terminus, X is hydrogen, and at the carboxyl terminus, Y is hydrogen or a metal, $R_1$ and $R_3$ are any amino acid residue, n is an integer between 1 and 1000, and $R_2$ is an amino acid sequence of the invention, particularly an amino acid sequence selected from the group set forth in the Sequence Listing. In the formula above $R_2$ is oriented so that its amino terminal residue is at the left, bound to $R_1$, and its carboxy terminal residue is at the right, bound to $R_3$. Any stretch of amino acid residues denoted by either R group, where R is greater than 1, may be either a heteropolymer or a homopolymer, preferably a heteropolymer.

A fragment is a variant polypeptide having an amino acid sequence that entirely is the same as part but not all of the amino acid sequence of the aforementioned polypeptides. As with polypeptides, fragments may be "free-standing," or comprised within a larger polypeptide of which they form a part or region, most preferably as a single continuous region, a single larger polypeptide.

Preferred fragments include, for example, truncation polypeptides having a portion of the amino acid sequence of the Sequence Listing, or of variants thereof, such as a continuous series of residues that includes the amino terminus, or a continuous series of residues that includes the carboxyl terminus. Degradation forms of the polypeptides of the invention in a host cell, particularly a *Staphylococcus aureus*, are also preferred. Further preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions.

Also preferred are biologically active fragments which are those fragments that mediate activities of polypeptides of the invention, including those with a similar activity or an improved activity, or with a decreased undesirable activity. Also included are those fragments that are antigenic or immunogenic in an animal, especially in a human. Particularly preferred are fragments comprising receptors or domains of enzymes that confer a function essential for viability of *Staphylococcus aureus* or the ability to initiate, or maintain cause disease in an individual, particularly a human.

Variants that are fragments of the polypeptides of the invention may be employed for producing the corresponding full-length polypeptide by peptide synthesis; therefore, these variants may be employed as intermediates for producing the full-length polypeptides of the invention.

In addition to the standard single and triple letter representations for amino acids, the term "X" or "Xaa" is also used. "X" and "Xaa" mean that any of the twenty naturally occuring amino acids may appear at such a designated position in the polypeptide sequence.

## Polynucleotides

Another aspect of the invention relates to isolated polynucleotides that encode the polypeptides of the invention having a deduced amino acid sequence selected from the group consisting of the sequences in the Sequence Listing and polynucleotides closely related thereto and variants thereof.

Using the information provided herein, such as the polynucleotide sequences set out in the Sequence Listing, a polynucleotide of the invention encoding polypeptide may be obtained using standard cloning and screening methods, such as those for cloning and sequencing chromosomal DNA fragments from bacteria using *Staphylococcus aureus* WCUH 29 cells as starting material, followed by obtaining a full length clone. For example, to obtain a polynucleotide sequence of the invention, such as a sequence set forth in the Sequence Listing, typically a library of clones of chromosomal DNA of *Staphylococcus aureus* WCUH 29 in *E.coli* or some other suitable host is probed with a radiolabeled oligonucleotide, preferably a 17-mer or longer, derived from a partial sequence. Clones carrying DNA identical to that of the probe can then be distinguished using stringent conditions. By sequencing the individual clones thus identified with sequencing primers designed from the original sequence it is then possible to extend the sequence in both directions to determine the full gene sequence. Conveniently, such sequencing is performed using denatured double stranded DNA prepared from a plasmid clone. Suitable techniques are described by Maniatis, T., Fritsch, E.F. and Sambrook et al., *MOLECULAR CLONING, A LABORATORY MANUAL,* 2nd Ed.; Cold Spring Harbor Laboratory Press, Cold Spring Harbor, New York (1989). (see in particular Screening By Hybridization 1.90 and Sequencing Denatured Double-Stranded DNA Templates 13.70). Illustrative of the invention, the polynucleotides set out in the Sequence Listing were discovered in a DNA library derived from *Staphylococcus aureus* WCUH 29.

The DNA sequences set out in the Sequence Listing each contains at least one open reading frame encoding a protein having at least about the number of amino acid residues set forth in the Sequence Listing. The start and stop codons of each open reading frame (herein "ORF") DNA are the first three and the last three nuclotides of each polynucleotide set forth in the Sequence Listing.

Certain polynucleotides and polypeptides of the invention are structurally related to known proteins as set forth in Table 1. These proteins exhibit greatest homology to the homologue listed in Table 1 from among the known proteins.

The invention provides a polynucleotide sequence identical over its entire length to each coding sequence in the Sequence Listing. Also provided by the invention is the coding sequence for the mature polypeptide or a fragment thereof, by itself as well as the coding sequence for the mature polypeptide or a fragment in reading frame with other coding sequence, such as those encoding a leader or secretory sequence, a pre-, or pro- or prepro- protein sequence. The polynucleotide may also contain non-coding sequences, including for example, but not limited to non-coding 5' and 3' sequences, such as the transcribed, non-translated sequences, termination signals, ribosome binding sites, sequences that stabilize mRNA, introns, polyadenylation signals, and additional coding sequence which encode additional amino acids. For example, a marker sequence that facilitates purification of the fused polypeptide can be encoded. In certain embodiments of the invention, the marker sequence is a hexa-histidine peptide, as provided in the pQE vector (Qiagen, Inc.) and described in Gentz *et al., Proc. Natl. Acad. Sci., USA 86:* 821-824 (1989), or an HA tag (Wilson *et al., Cell 37:* 767 (1984). Polynucleotides of the invention also include, but are not limited to, polynucleotides comprising a structural gene and its naturally associated sequences that control gene expression.

The invention also includes polynucleotides of the formula:

$$X\text{-}(R_1)_n\text{-}(R_2)\text{-}(R_3)_n\text{-}Y$$

wherein, at the 5' end of the molecule, X is hydrogen, and at the 3' end of the molecule, Y is hydrogen or a metal, $R_1$ and $R_3$ is any nucleic acid residue, n is an integer between 1 and 3000, and $R_2$ is a nucleic acid sequence of the invention, particularly a nucleic acid sequence selected from the group set forth in the Sequence Listing. In the polynucleotide formula above $R_2$ is oriented so that its 5' end residue is at the left, bound to $R_1$, and its 3' end residue is at the right, bound to $R_3$. Any stretch of nucleic acid residues denoted by either R group, where R is greater than 1, may be either a heteropolymer or a homopolymer, preferably a heteropolymer. In a preferred embodiment n is an integer between 1 and 1000

The term "polynucleotide encoding a polypeptide" as used herein encompasses polynucleotides that include a sequence encoding a polypeptide of the invention, particularly a bacterial polypeptide and more particularly a polypeptide of the *Staphylococcus aureus* having an amino acid sequence set out in the Sequence Listing. The term also encompasses polynucleotides that include a single continuous region or discontinuous regions encoding the polypeptide (for example, interrupted by integrated phage or an insertion sequence or editing) together with additional regions, that also may contain coding and/or non-coding sequences.

The invention further relates to variants of the polynucleotides described herein that encode for variants of the polypeptide having the deduced amino acid sequence of the Sequence Listing. Variants that are fragments of the polynucleotides of the invention may be used to synthesize full-length polynucleotides of the invention.

Further particularly preferred embodiments are polynucleotides encoding polypeptide variants, that have the amino acid sequence of a polypeptide of the Sequence Listing in which several, a few, 5 to 10, 1 to 5, 1 to 3, 2, 1 or no amino acid residues are substituted, deleted or added, in any combination. Especially preferred among these are silent substitutions, additions and deletions, that do not alter the properties and activities of such polynucleotide.

Further preferred embodiments of the invention are polynucleotides that are at least 50%, 60% or 70% identical over their entire length to a polynucleotide encoding a polypeptide having the amino acid sequence set out in the Sequence Listing, and polynucleotides that are complementary to such polynucleotides. Alternatively, most highly preferred are polynucleotides that comprise a region that is at least 80% identical over its entire length to a polynucleotide encoding a polypeptide of the deposited strain and polynucleotides complementary thereto. In this regard, polynucleotides at least 90% identical over their entire length to the same are particularly preferred, and among these particularly preferred polynucleotides, those with at least 95% are especially preferred. Furthermore, those with at least 97% are highly preferred among those with at least 95%, and among these those with at least 98% and at least 99% are particularly highly preferred, with at least 99% being the more preferred.

A preferred embodiment is an isolated polynucleotide comprising a polynucleotide sequence selected from the group consisting of: a polynucleotide having at least a 50% identity to a polynucleotide encoding a polypeptide comprising the amino acid sequence of the Sequence Listing and obtained from a prokaryotic species other than *Staphylococcus aureus*; and a polynucleotide encoding a polypeptide comprising an amino acid sequence which is at least 50% identical to the amino acid sequence of the Sequence Listing and obtained from a prokaryotic species other than *Staphylococcus aureus.*

Preferred embodiments are polynucleotides that encode polypeptides that retain substantially the same biological function or activity as the mature polypeptide encoded by the DNA of the Sequence Listing.

The invention further relates to polynucleotides that hybridize to the herein above-described sequences. In this regard, the invention especially relates to polynucleotides that hybridize under stringent conditions to the herein above-described polynucleotides. As herein used, the terms "stringent conditions" and "stringent hybridization conditions" mean hybridization will occur only if there is at least 95% and preferably at least 97% identity between the sequences. An example of stringent hybridization conditions is overnight incubation at 42°C in a solution comprising: 50% formamide, 5x SSC (150mM NaCl, 15mM trisodium citrate), 50 mM sodium phosphate (pH7.6), 5x Denhardt's solution, 10% dextran sulfate, and 20 micrograms/ml denatured, sheared salmon sperm DNA, followed by washing the hybridization support in 0.1x SSC at about 65°C. Hybridization and wash conditions are well known and exemplified in Sambrook, *et al.,* Molecular Cloning: A Laboratory Manual, Second Edition, Cold Spring Harbor, N.Y., (1989), particularly Chapter 11 therein.

The invention also provides a polynucleotide consisting essentially of a polynucleotide sequence obtainable by screening an appropriate library containing the complete gene for a polynucleotide sequence set forth in the Sequence Listing under stringent hybridization conditions with a probe having the sequence of said polynucleotide sequence or a fragment thereof; and isolating said DNA sequence. Fragments useful for obtaining such a polynucleotide include, for example, probes and primers described elsewhere herein.

As discussed additionally herein regarding polynucleotide assays of the invention, for instance, polynucleotides of the invention as discussed above, may be used as a hybridization probe for RNA, cDNA and genomic DNA to isolate full-length cDNAs and genomic clones encoding a polypeptide and to isolate cDNA and genomic clones of other genes that have a high sequence similarity to a polynucleotide set forth in the Sequence Listing. Such probes generally will comprise at least 15 bases. Preferably, such probes will have at least 30 bases and may have at least 50 bases. Particularly preferred probes will have at least 30 bases and will have 50 bases or less.

For example, the coding region of each gene that comprises or is comprised by a polynucleotide set forth in the Sequence Listing may be isolated by screening using a DNA sequence provided in the Sequence Listing to synthesize an oligonucleotide probe. A labeled oligonucleotide having a sequence complementary to that of a gene of the invention is then used to screen a library of cDNA, genomic DNA or mRNA to determine which members of the library the probe hybridizes to.

The polynucleotides and polypeptides of the invention may be employed, for example, as research reagents and materials for discovery of treatments of and diagnostics for disease, particularly human disease, as further discussed herein relating to polynucleotide assays.

Polynucleotides of the invention that are oligonucleotides derived from the a polynucleotide or polypeptide sequence set forth in the Sequence Listing may be used in the processes herein as described, but preferably for PCR, to determine whether or not the polynucleotides identified herein in whole or in part are transcribed in bacteria in infected tissue. It is recognized that such sequences will also have utility in diagnosis of the stage of infection and type of infection the pathogen has attained.

The invention also provides polynucleotides that may encode a polypeptide that is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may allow protein transport, may lengthen or shorten protein half-life or may facilitate manipulation of a protein for assay or production, among other things. As generally is the case *in vivo,* the additional amino acids may be processed away from the mature protein by cellular enzymes.

A precursor protein, having the mature form of the polypeptide fused to one or more prosequences may be an inactive form of the polypeptide. When prosequences are removed such inactive precursors generally are activated. Some or all of the prosequences may be removed before activation. Generally, such precursors are called proproteins.

In addition to the standard A, G, C, T/U representations for nucleic acid bases, the term "N" is also used. "N" means that any of the four DNA or RNA bases may appear at such a designated position in the DNA or RNA sequence, except it is preferred that N is not a base that when taken in combination with adjacent nucleotide positions, when read in the correct reading frame, would have the effect of generating a premature termination codon in such reading frame.

In sum, a polynucleotide of the invention may encode a mature protein, a mature protein plus a leader sequence (which may be referred to as a preprotein), a precursor of a mature protein having one or more prosequences that are not the leader sequences of a preprotein, or a preproprotein, which is a precursor to a proprotein, having a leader sequence and one or more prosequences, which generally are removed during processing steps that produce active and mature forms of the polypeptide.

## Vectors, host cells, expression

The invention also relates to vectors that comprise a polynucleotide or polynucleotides of the invention, host cells that are genetically engineered with vectors of the invention and the production of polypeptides of the invention by recombinant techniques. Cell-free translation systems can also be employed to produce such proteins using RNAs derived from the DNA constructs of the invention.

For recombinant production, host cells can be genetically engineered to incorporate expression systems or portions thereof or polynucleotides of the invention. Introduction of a polynucleotide into the host cell can be effected by methods described in many standard laboratory manuals, such as Davis et al., *BASIC METHODS IN MOLECULAR BIOLOGY,* (1986) and Sambrook et al., *MOLECULAR CLONING: A LABORATORY MANUAL,* 2nd Ed., Cold Spring Harbor Laboratory Press, Cold Spring Harbor, N.Y. (1989), such as, calcium phosphate transfection, DEAE-dextran mediated transfection, transvection, microinjection, cationic lipid-mediated transfection, electroporation, transduction, scrape loading, ballistic introduction and infection.

Representative examples of appropriate hosts include bacterial cells, such as streptococci, staphylococci, enterococci *E. coli,* streptomyces and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, 293 and Bowes melanoma cells; and plant cells.

A great variety of expression systems can be used to produce the polypeptides of the invention. Such vectors include, among others, chromosomal, episomal and virus-derived vectors, *e.g.,* vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques, such as, for example, those set forth in Sambrook *et al., MOLECULAR CLONING, A LABORATORY MANUAL, (supra).*

For secretion of the translated protein into the lumen of the endoplasmic reticulum, into the periplasmic space or into the extracellular environment, appropriate secretion signals may be incorporated into the expressed polypeptide. These signals may be endogenous to the polypeptide or they may be heterologous signals.

Polypeptides of the invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography, and lectin chromatography. Most preferably, high performance liquid chromatography is employed for purification. Well known techniques for refolding protein may be employed to regenerate active conformation when the polypeptide is denatured during isolation and or purification.

**Diagnostic Assays**

This invention is also related to the use of the polynucleotides of the invention for use as diagnostic reagents. Detection of such polynucleotides in a eukaryote, particularly a mammal, and especially a human, will provide a diagnostic method for diagnosis of a disease. Eukaryotes (herein also "individual(s)"), particularly mammals, and especially humans, infected with an organism comprising a gene of the invention may be detected at the nucleic acid level by a variety of techniques.

Nucleic acids for diagnosis may be obtained from an infected individual's cells and tissues, such as bone, blood, muscle, cartilage, and skin. Genomic DNA may be used directly for detection or may be amplified enzymatically by using PCR or other amplification technique prior to analysis. RNA or cDNA may also be used in the same ways. Using amplification, characterization of the species and strain of prokaryote present in an individual, may be made by an analysis of the genotype of the prokaryote gene. Deletions and insertions can be detected by a change in size of the amplified product in comparison to the genotype of a reference sequence. Point mutations can be identified by hybridizing amplified DNA to labeled polynucleotide sequences of the invention. Perfectly matched sequences can be distinguished from mismatched duplexes by RNase digestion or by differences in melting temperatures. DNA sequence differences may also be detected by alterations in the electrophoretic mobility of the DNA fragments in gels, with or without denaturing agents, or by direct DNA sequencing. See, *e.g.,* Myers et al., *Science, 230:* 1242 (1985). Sequence changes at specific locations also may be revealed by nuclease protection assays, such as RNase and S1 protection or a chemical cleavage method. See, *e.g.,* Cotton et al., *Proc. Natl. Acad. Sci., USA, 85:* 4397-4401 (1985).

Cells carrying mutations or polymorphisms in the gene of the invention may also be detected at the DNA level by a variety of techniques, to allow for serotyping, for example. For example, RT-PCR can be used to detect mutations. It is particularly preferred to used RT-PCR in conjunction with automated detection systems, such as, for example, GeneScan. RNA or CDNA may also be used for the same purpose, PCR or RT-PCR. As an example, PCR primers complementary to a nucleic acid encoding a polypeptide of the invention can be used to identify and analyze mutations. These primers may be used for, among other things, amplifying a DNA of the invention isolated from a sample derived from an individual. The primers may be used to amplify the gene isolated from an infected individual such that the gene may then be subject to various techniques for elucidation of the DNA sequence. In this way, mutations in the DNA sequence may be detected and used to diagnose infection and to serotype and/or classify the infectious agent.

The invention further provides a process for diagnosing, disease, preferably bacterial infections, more preferably infections by *Staphylococcus aureus,* and most preferably disease, such as, infections of the upper respiratory tract (e.g., otitis media, bacterial tracheitis, acute epiglottitis, thyroiditis), lower respiratory (e.g., empyema, lung abscess), cardiac (e.g., infective endocarditis), gastrointestinal (e.g., secretory diarrhoea, splenic absces, retroperitoneal abscess), CNS (e.g., cerebral abscess), eye (e.g., blepharitis, conjunctivitis, keratitis, endophthalmitis, preseptal and orbital cellulitis, darcryocystitis), kidney and urinary tract (e.g., epididymitis, intrarenal and perinephric abscess, toxic shock syndrome), skin (e.g., impetigo, folliculitis, cutaneous abscesses, cellulitis, wound infection, bacterial myositis) bone and joint (e.g., septic arthritis, osteomyelitis), comprising determining from a sample derived from an individual a increased level of expression of polynucleotide having the sequence of the Sequence Listing. Increased or decreased expression of a polynucleotide of the invention can be measured using any on of the methods well known in the art for the quantitation of polynucleotides, such as, for example, amplification, PCR, RT-PCR, RNase protection, Northern blotting and other hybridization methods.

In addition, a diagnostic assay in accordance with the invention for detecting over-expression of a polypeptide of the invention compared to normal control tissue samples may be used to detect the presence of an infection, for example. Assay techniques that can be used to determine levels of a protein, in a sample derived from a host are well-known to those of skill in the art. Such assay methods include radioimmunoassays, competitive-binding assays, Western Blot analysis and ELISA assays.

**Antibodies**

The polypeptides of the invention or variants thereof or cells expressing them can be used as an immunogen to produce antibodies immunospecific for such polypeptides. "Antibodies" as used herein includes monoclonal and polyclonal antibodies, chimeric, single chain, simianized antibodies and humanized antibodies, as well as Fab fragments, including the products of an Fab immunolglobulin expression library.

Antibodies generated against the polypeptides of the invention can be obtained by administering the polypeptides or epitope-bearing fragments, analogues or cells to an animal, preferably a nonhuman, using routine protocols. For preparation of monoclonal antibodies, any technique known in the art that provides antibodies produced by continuous cell line cultures can be used. Examples include various techniques, such as those in Kohler, G. and Milstein, C., *Nature 256:* 495-497 (1975); Kozbor *et al., Immunology Today 4:* 72 (1983); Cole et al., pg. 77-96 in *MONOCLONAL ANTIBODIES AND CANCER THERAPY,* Alan R. Liss, Inc. (1985).

Techniques for the production of single chain antibodies (U.S. Patent No. 4,946,778) can be adapted to produce single chain antibodies to polypeptides of this invention. Also, transgenic mice, or other organisms such as other mammals, may be used to express humanized antibodies.

Alternatively phage display technology may be utilized to select antibody genes with binding activities towards the polypeptide either from repertoires of PCR amplified v-genes of lymphocytes from humans screened for possessing recognition of a polypeptide of the invention or from naive libraries (McCafferty, J. et al., (1990), Nature **348**, 552-554; Marks, J. et al., (1992) Biotechnology *10*, 779-783). The affinity of these antibodies can also be improved by chain shuffling (Clackson, T. et al., (1991) Nature **352**, 624-628).

If two antigen binding domains are present each domain may be directed against a different epitope - termed 'bispecific' antibodies.

The above-described antibodies may be employed to isolate or to identify clones expressing the polypeptides to purify the polypeptides by affinity chromatography.

Thus, among others, antibodies against a polypeptide of the invention may be employed to treat infections, particularly bacterial infections and especially disease, such as, infections of the upper respiratory tract (e.g., otitis media, bacterial tracheitis, acute epiglottitis, thyroiditis), lower respiratory (e.g., empyema, lung abscess), cardiac (e. g., infective endocarditis), gastrointestinal (e.g., secretory diarrhoea, splenic absces, retroperitoneal abscess), CNS (e.g., cerebral abscess), eye (e.g., blepharitis, conjunctivitis, keratitis, endophthalmitis, preseptal and orbital cellulitis, darcryocystitis), kidney and urinary tract (e.g., epididymitis, intrarenal and perinephric abscess, toxic shock syndrome), skin (e.g., impetigo, folliculitis, cutaneous abscesses, cellulitis, wound infection, bacterial myositis) bone and joint (e. g., septic arthritis, osteomyelitis).

Polypeptide variants include antigenically, epitopically or immunologically equivalent variants that form a particular aspect of this invention. The term "antigenically equivalent derivative" as used herein encompasses a polypeptide or its equivalent which will be specifically recognized by certain antibodies which, when raised to the protein or polypeptide according to the invention, interfere with the immediate physical interaction between pathogen and mammalian host. The term "immunologically equivalent derivative" as used herein encompasses a peptide or its equivalent which when used in a suitable formulation to raise antibodies in a vertebrate, the antibodies act to interfere with the immediate physical interaction between pathogen and mammalian host.

The polypeptide, such as an antigenically or immunologically equivalent derivative or a fusion protein thereof is used as an antigen to immunize a mouse or other animal such as a rat or chicken. The fusion protein may provide stability to the polypeptide. The antigen may be associated, for example by conjugation, with an immunogenic carrier protein for example bovine serum albumin (BSA) or keyhole limpet haemocyanin (KLH). Alternatively a multiple antigenic peptide comprising multiple copies of the protein or polypeptide, or an antigenically or immunologically equivalent polypeptide thereof may be sufficiently antigenic to improve immunogenicity so as to obviate the use of a carrier.

Preferably, the antibody or variant thereof is modified to make it less immunogenic in the individual. For example, if the individual is human the antibody may most preferably be "humanized"; where the complimentarity determining region(s) of the hybridoma-derived antibody has been transplanted into a human monoclonal antibody , for example as described in Jones, P. et al. (1986), *Nature 321,* 522-525 or Tempest et al.,(1991) *Biotechnology 9,* 266-273.

The use of a polynucleotide of the invention in genetic immunization will preferably employ a suitable delivery method such as direct injection of plasmid DNA into muscles (Wolff et al., Hum Mol Genet 1992, 1:363, Manthorpe et al., Hum. Gene Ther. 1963:4, 419), delivery of DNA complexed with specific protein carriers (Wu et al., J Biol Chem. 1989: 264,16985), coprecipitation of DNA with calcium phosphate (Benvenisty & Reshef, PNAS, 1986:83,9551), encapsulation of DNA in various forms of liposomes (Kaneda et al., Science 1989:243,375), particle bombardment (Tang et al., Nature 1992, 356:152, Eisenbraun et al., DNA Cell Biol 1993, 12:791) and *in vivo* infection using cloned retroviral vectors (Seeger et al., PNAS 1984:81,5849).

## Antagonists and agonists - assays and molecules

Polypeptides of the invention may also be used to assess the binding of small molecule substrates and ligands in, for example, cells, cell-free preparations, chemical libraries, and natural product mixtures. These substrates and ligands may be natural substrates and ligands or may be structural or functional mimetics. See, *e.g.,* Coligan *et al., Current Protocols in Immunology 1(2):* Chapter 5 (1991).

The invention also provides a method of screening compounds to identify those which enhance (agonist) or block (antagonist) the action of a polypeptides or polynucleotides of the invention, particularly those compounds that are bacteriostatic and/or bacteriocidal. The method of screening may involve high-throughput techniques. For example, to screen for agonists or antagoists, a synthetic reaction mix, a cellular compartment, such as a membrane, cell envelope or cell wall, or a preparation of any thereof, comprising a polypeptide of the invention and a labeled substrate or ligand of such polypeptide is incubated in the absence or the presence of a candidate molecule that may be an agonist or antagonist of a polypeptide of the invention. The ability of the candidate molecule to agonize or antagonize a polypeptide of the invention is reflected in decreased binding of the labeled ligand or decreased production of product from such substrate. Molecules that bind gratuitously, *i.e.,* without inducing the effects of a polypeptide of the invention are most likely to be good antagonists. Molecules that bind well and increase the rate of product production from substrate are agonists. Detection of the rate or level of production of product from substrate may be enhanced by using a reporter system. Reporter systems that may be useful in this regard include but are not limited to colorimetric labeled substrate converted into product, a reporter gene that is responsive to changes in polynucleotide or polypeptide activity, and binding assays known in the art.

Another example of an assay for antagonists of polypeptides of the invention is a competitive assay that combines any such polypeptide and a potential antagonist with a compound which binds such polypeptide, natural substrates or ligands, or substrate or ligand mimetics, under appropriate conditions for a competitive inhibition assay. A polypeptide of the invention can be labeled, such as by radioactivity or a colorimetric compound, such that the number of such polypeptide molecules bound to a binding molecule or converted to product can be determined accurately to assess the effectiveness of the potential antagonist.

Potential antagonists include small organic molecules, peptides, polypeptides and antibodies that bind to a polynucleotide or polypeptide of the invention and thereby inhibit or extinguish its activity. Potential antagonists also may be small organic molecules, a peptide, a polypeptide such as a closely related protein or antibody that binds the same sites on a binding molecule, such as a binding molecule, without inducing activities induced by a polypeptide of the invention, thereby preventing the action of such polypeptide by excluding it from binding.

Potential antagonists include a small molecule that binds to and occupies the binding site of the polypeptide thereby preventing binding to cellular binding molecules, such that normal biological activity is prevented. Examples of small molecules include but are not limited to small organic molecules, peptides or peptide-like molecules. Other potential antagonists include antisense molecules (see Okano, *J. Neurochem. 56:* 560 (1991); *OLIGODEOXYNUCLEOTIDES AS ANTISENSE INHIBITORS OF GENE EXPRESSION,* CRC Press, Boca Raton, FL (1988), for a description of these molecules). Preferred potential antagonists include compounds related to and variants of a polypeptide of the invention.

Each of the DNA sequences provided herein may be used in the discovery and development of antibacterial compounds. The encoded protein, upon expression, can be used as a target for the screening of antibacterial drugs. Additionally, the DNA sequences encoding the amino terminal regions of the encoded protein or Shine-Delgarno or other translation facilitating sequences of the respective mRNA can be used to construct antisense sequences to control the expression of the coding sequence of interest.

The invention also provides the use of the polypeptide, polynucleotide or inhibitor of the invention to interfere with the initial physical interaction between a pathogen and mammalian host responsible for sequelae of infection. In particular the molecules of the invention may be used: in the prevention of adhesion of bacteria, in particular gram positive bacteria, to mammalian extracellular matrix proteins on in-dwelling devices or to extracellular matrix proteins in wounds; to block protein-mediated mammalian cell invasion by, for example, initiating phosphorylation of mammalian tyrosine kinases (Rosenshine *et al., Infect. Immun.* 60:2211 (1992); to block bacterial adhesion between mammalian extracellular matrix proteins and bacterial proteins that mediate tissue damage and; to block the normal progression of pathogenesis in infections initiated other than by the implantation of in-dwelling devices or by other surgical techniques.

The antagonists and agonists of the invention may be employed, for instance, to inhibit and treat disease, such as, infections of the upper respiratory tract (e.g., otitis media, bacterial tracheitis, acute epiglottitis, thyroiditis), lower respiratory (e.g., empyema, lung abscess), cardiac (e.g., infective endocarditis), gastrointestinal (e.g., secretory diarrhoea, splenic absces, retroperitoneal abscess), CNS (e.g., cerebral abscess), eye (e.g., blepharitis, conjunctivitis, keratitis, endophthalmitis, preseptal and orbital cellulitis, darcryocystitis), kidney and urinary tract (e.g., epididymitis, intrarenal and perinephric abscess, toxic shock syndrome), skin (e.g., impetigo, folliculitis, cutaneous abscesses, cellulitis, wound infection, bacterial myositis) bone and joint (e.g., septic arthritis, osteomyelitis).

*Helicobacter pylori* (herein *H. pylori)* bacteria infect the stomachs of over one-third of the world's population causing stomach cancer, ulcers, and gastritis (International Agency for Research on Cancer (1994) Schistosomes, Liver Flukes and Helicobacter Pylori (International Agency for Research on Cancer, Lyon, France; http://www.uicc.ch/ecp/ecp2904.htm). Moreover, the international Agency for Research on Cancer recently recognized a cause-and-effect relationship between *H. pylori* and gastric adenocarcinoma, classifying the bacterium as a Group I (definite) carcinogen. Preferred antimicrobial compounds of the invention found using screens provided by the invention, particularly broad-spectrum antibiotics, should be useful in the treatment of *H. pylori* infection. Such treatment should decrease the advent of *H.* pylori-induced cancers, such as gastrointestinal carcinoma. Such treatment should also cure gastric ulcers and gastritis.

**Vaccines**

Another aspect of the invention relates to a method for inducing an immunological response in an individual, particularly a mammal which comprises inoculating the individual with a polypeptide of the invention, or a fragment or variant thereof, adequate to produce antibody and/ or T cell immune response to protect said individual from infection, particularly bacterial infection and most particularly *Staphylococcus aureus* infection. Also provided are methods whereby such immunological response slows bacterial replication. Yet another aspect of the invention relates to a method of inducing immunological response in an individual which comprises delivering to such individual a nucleic acid vector to direct expression of a polynucleotide or polypeptide of the invention, or a fragment or a variant thereof, for expressing such polynucleotide or polypeptide, or a fragment or a variant thereof *in vivo* in order to induce an immunological response, such as, to produce antibody and/ or T cell immune response, including, for example, cytokine-producing T cells or cytotoxic T cells, to protect said individual from disease, whether that disease is already established within the individual or not. One way of administering the gene is by accelerating it into the desired cells as a coating on particles or otherwise.

Such nucleic acid vector may comprise DNA, RNA, a modified nucleic acid, or a DNA/RNA hybrid.

A further aspect of the invention relates to an immunological composition which, when introduced into an individual capable or having induced within it an immunological response, induces an immunological response in such individual to a polynucleotide of the invention or protein coded therefrom, wherein the composition comprises a recombinant polynucleotide or protein coded therefrom comprising DNA which codes for and expresses an antigen of said polynucleotide or protein coded therefrom. The immunological response may be used therapeutically or prophylactically and may take the form of antibody immunity or cellular immunity such as that arising from CTL or CD4+ T cells.

A polypeptide of the invention or a fragment thereof may be fused with co-protein which may not by itself produce antibodies, but is capable of stabilizing the first protein and producing a fused protein which will have immunogenic and protective properties. Thus fused recombinant protein, preferably further comprises an antigenic co-protein, such as lipoprotein D from *Hemophilus influenzae,* Glutathione-S-transferase (GST) or beta-galactosidase, relatively large co-proteins which solubilize the protein and facilitate production and purification thereof. Moreover, the co-protein may act as an adjuvant in the sense of providing a generalized stimulation of the immune system. The co-protein may be attached to either the amino or carboxy terminus of the first protein.

Provided by this invention are compositions, particularly vaccine compositions, and methods comprising the polypeptides or polynucleotides of the invention and immunostimulatory DNA sequences, such as those described in Sato, Y. *et al.* Science 273: 352 (1996).

Also, provided by this invention are methods using the described polynucleotide or particular fragments thereof which have been shown to encode non-variable regions of bacterial cell surface proteins in DNA constructs used in such genetic immunization experiments in animal models of infection with *Staphylococcus aureus* will be particularly useful for identifying protein epitopes able to provoke a prophylactic or therapeutic immune response. It is believed that this approach will allow for the subsequent preparation of monoclonal antibodies of particular value from the requisite organ of the animal successfully resisting or clearing infection for the development of prophylactic agents or therapeutic treatments of bacterial infection, particularly *Staphylococcus aureus* infection, in mammals, particularly humans.

The polypeptide may be used as an antigen for vaccination of a host to produce specific antibodies which protect against invasion of bacteria, for example by blocking adherence of bacteria to damaged tissue. Examples of tissue damage include wounds in skin or connective tissue caused, e.g., by mechanical, chemical or thermal damage or by implantation of indwelling devices, or wounds in the mucous membranes, such as the mouth, mammary glands, urethra or vagina.

The invention also includes a vaccine formulation which comprises an immunogenic recombinant protein of the invention together with a suitable carrier. Since the protein may be broken down in the stomach, it is preferably administered parenterally, including, for example, administration that is subcutaneous, intramuscular, intravenous, or intradermal. Formulations suitable for parenteral administration include aqueous and non-aqueous sterile injection solutions which may contain anti-oxidants, buffers, bacteriostats and solutes which render the formulation isotonic with the bodily fluid, preferably the blood, of the individual; and aqueous and non-aqueous sterile suspensions which may include suspending agents or thickening agents. The formulations may be presented in unit-dose or multi-dose containers, for example, sealed ampules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art.

The dosage will depend on the specific activity of the vaccine and can be readily determined by routine experimentation.

While the invention has been described with reference to certain protein, such as, for example, those set forth in the Sequence Listing, it is to be understood that this covers fragments of the naturally occurring protein and similar proteins with additions, deletions or substitutions which do not substantially affect the immunogenic properties of the recombinant protein.

## Compositions, kits and administration

The invention also relates to compositions comprising the polynucleotide or the polypeptides discussed above or their agonists or antagonists. The polypeptides of the invention may be employed in combination with a non-sterile or sterile carrier or carriers for use with cells, tissues or organisms, such as a pharmaceutical carrier suitable for administration to a subject. Such compositions comprise, for instance, a media additive or a therapeutically effective amount of a polypeptide of the invention and a pharmaceutically acceptable carrier or excipient. Such carriers may include, but are not limited to, saline, buffered saline, dextrose, water, glycerol, ethanol and combinations thereof. The formulation should suit the mode of administration. The invention further relates to diagnostic and pharmaceutical packs and kits comprising one or more containers filled with one or more of the ingredients of the aforementioned compositions of the invention.

Polypeptides and other compounds of the invention may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal or intradermal routes among others.

In therapy or as a prophylactic, the active agent may be administered to an individual as an injectable composition, for example as a sterile aqueous dispersion, preferably isotonic.

Alternatively the composition may be formulated for topical application for example in the form of ointments, creams, lotions, eye ointments, eye drops, ear drops, mouthwash, impregnated dressings and sutures and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

For administration to mammals, and particularly humans, it is expected that the daily dosage level of the active agent will be from 0.01 mg/kg to 10 mg/kg, typically around 1 mg/kg. The physician in any event will determine the actual dosage which will be most suitable for an individual and will vary with the age, weight and response of the particular individual. The above dosages are exemplary of the average case. There can, of course, be individual instances where higher or lower dosage ranges are merited, and such are within the scope of this invention.

In-dwelling devices include surgical implants, prosthetic devices and catheters, i.e., devices that are introduced to the body of an individual and remain in position for an extended time. Such devices include, for example, artificial joints, heart valves, pacemakers, vascular grafts, vascular catheters, cerebrospinal fluid shunts, urinary catheters, continuous ambulatory peritoneal dialysis (CAPD) catheters.

The composition of the invention may be administered by injection to achieve a systemic effect against relevant bacteria shortly before insertion of an in-dwelling device. Treatment may be continued after surgery during the in-body time of the device. In addition, the composition could also be used to broaden perioperative cover for any surgical technique to prevent bacterial wound infections, especially *Staphylococcus aureus* wound infections.

Many orthopedic surgeons consider that humans with prosthetic joints should be considered for antibiotic prophylaxis before dental treatment that could produce a bacteremia. Late deep infection is a serious complication sometimes leading to loss of the prosthetic joint and is accompanied by significant morbidity and mortality. It may therefore be possible to extend the use of the active agent as a replacement for prophylactic antibiotics in this situation.

In addition to the therapy described above, the compositions of this invention may be used generally as a wound treatment agent to prevent adhesion of bacteria to matrix proteins exposed in wound tissue and for prophylactic use in dental treatment as an alternative to, or in conjunction with, antibiotic prophylaxis.

Alternatively, the composition of the invention may be used to bathe an indwelling device immediately before insertion. The active agent will preferably be present at a concentration of 1μg/ml to 10mg/ml for bathing of wounds or indwelling devices.

A vaccine composition is conveniently in injectable form. Conventional adjuvants may be employed to enhance the immune response. A suitable unit dose for vaccination is 0.5-5 microgram/kg of antigen, and such dose is preferably administered 1-3 times and with an interval of 1-3 weeks. With the indicated dose range, no adverse toxicological effects will be observed with the compounds of the invention which would preclude their administration to suitable individuals.

Each reference disclosed herein is incorporated by reference herein in its entirety. Any patent application to which this application claims priority is also incorporated by reference herein in its entirety.

**TABLES**

Certain pertinent data for each of the polypeptides and polynucleotides set forth in the Sequence Listing are summarized in the following Table.

**<u>TABLE 1</u>**

Provided in this Table is the closest homologue of each polypeptide encoded by each ORF of the invention based on a comparison of the sequences of in the Sequence Listing with sequences available in the public domain (see, the columen labeled "Homologue identification"). Preferred polypeptides ("PPs") encoded by the ORFs of the invention, particularly full length proteins either obtained using such ORFs or encoded entirely by such ORFs, are ones that have a biological function of the homologue listed, among other functions. The analysis used to determine each homologue listed in Table 1 was either BlastP, BlastX or MPSearch, each of which is well known. The left-most column of the table shows the relationship between each polynucleotide ("PN") of the invention and a deduced polypeptide ("PP") that it encodes. The column labeled "primer pair" shows the left and right primers used to amplify the polynucleotide in the left-most column. These primers may also be used to show *in vivo* expression or each gene, using one of the mouse models described herein, for example. The right-most column shows the detection of an expression product from the polynucleotide and the infection model in which it was detected.

| Related PN and PP SEQ ID NOS: | Primer pair SEQ ID NOS: (left/right) | Homologue identification | in vivo expression |
|---|---|---|---|
| 1/11 | 21/22 | sp\|P37580\|FHUD_BACSU FERRICHROME-BINDING PROTEIN PRECURSOR | 3day groin positive<br><br>8day pyelonephritis positive |
| 2/12 | 23/24 | gi\|1881232 (AB001488) PROBABLE ACETYLTRANSFERASE. [Bacillus subtilis] | 3day groin positive<br><br>8day pyelonephritis positive |
| 3/13 | 25/26 | gi\|1405336 (D86240) apparent membrane transporter [Staphylococcus aureus] | 3day groin positive<br><br>8day pyelonephritis positive |

| | | | |
|---|---|---|---|
| 4/14 | 27/28 | gi\|1001334 (D64006) sensory transduction histidine kinase [Synechocystis sp.] | 3day groin positive<br><br>8day pyelonephritis positive |
| 5/15 | 29/30 | sp\|P42020\|PEPT_LACLC PEPTIDASE T (aminotripeptidase) (tripeptidase) | 3day groin positive<br><br>8day pyelonephritis positive |
| 6/16 41/45 | 31/32 | gi\|1742360 (D90784) Phosphinothricin acetyltransferase (EC 2.3.1.-). | 3day groin positive<br><br>8day pyelonephritis positive |
| 7/17 42/46 | 33/34 | sp\|P50849\|PNP_BACSU POLYRIBONUCLEOTIDE NUCLEOTIDYLTRANSFE RASE (polynucleotide phosphorylase) (PNPASE) | 3day groin positive<br><br>8day pyelonephritis positive |

| | | | |
|---|---|---|---|
| 8/18 43/47 | 35/36 | gnl\|PID\|e281579 (Z82044) unidentified transporter-ATP binding [Bacillus subtilis] | 3day groin positive<br><br>8day pyelonephritis positive |
| 9/19 44/48 | 37/38 | sp\|P54182\|YPOP_BACSU apparent transcriptional regulator | 3day groin positive<br><br>8day pyelonephritis positive |
| 10/20 | 39/40 | gi\|576655 (U17054) ORF1 [Vibrio anguillarum] | 3day groin positive<br><br>8day pyelonephritis positive |

### EXAMPLES

The examples below are carried out using standard techniques, which are well known and routine to those of skill in the art, except where otherwise described in detail. The examples are illustrative, but do not limit the invention.

### Example 1 Strain selection, Library Production and Sequencing

Each polynucleotide having a DNA sequence given in the Sequence Listing was obtained from a library of clones of chromosomal DNA of *Staphylococcus aureus* in *E. coli.* The sequencing data from two or more clones containing overlapping *Staphylococcus aureus* DNAs was used to construct the contiguous DNA sequence in the Sequence Listing. Libraries may be prepared by routine methods, for example:
Methods 1 and 2 below.
Total cellular DNA is isolated from *Staphylococcus aureus* WCUH 29 according to standard procedures and size-fractionated by either of two methods.

### Method 1

Total cellular DNA is mechanically sheared by passage through a needle in order to size-fractionate according to standard procedures. DNA fragments of up to 11kbp in size are rendered blunt by treatment with exonuclease and DNA polymerase, and EcoRI linkers added. Fragments are ligated into the vector Lambda ZapII that has been cut with EcoRI, the library packaged by standard procedures and *E.coli* infected with the packaged library. The library is amplified by standard procedures.

### Method 2

Total cellular DNA is partially hydrolyzed with a one or a combination of restriction enzymes appropriate to generate a series of fragments for cloning into library vectors (e.g., RsaI, PaII, AluI, BshI235I), and such fragments are size-fractionated according to standard procedures. EcoRI linkers are ligated to the DNA and the fragments then ligated into the vector Lambda ZapII that have been cut with EcoRI, the library packaged by standard procedures, and *E.coli* infected with the packaged library. The library is amplified by standard procedures.

### Example 2 The determination of expression during infection of a gene from *Staphylococcus aureus*

Necrotic fatty tissue from an acute four day groin infection or a chronic 8 day pyelonephritis infection of *Staphylococcus aureus* WCUH29 in the mouse is efficiently disrupted and processed in the presence of chaotropic agents and RNAase inhibitor to provide a mixture of animal and bacterial RNA. The optimal conditions for disruption and processing to give stable preparations and high yields of bacterial RNA are followed by the use of hybridisation to a radiolabelled oligonucleotide specific to *Staphylococcus aureus* 16S RNA on Northern blots. The RNAase free, DNAase free, DNA and protein free preparations of RNA obtained are suitable for Reverse Transcription PCR (RT-PCR) using unique primer pairs designed from the sequence of each gene of *Staphylococcus aureus* WCUH29.

### a) Isolation of tissue infected with *Staphylococcus aureus* WCUH29 from a mouse animal model of an acute infection

10 ml. volumes of sterile nutrient broth (No.2 Oxoid) are seeded with isolated, individual colonies of *Staphylococcus aureus* WCUH29 from an agar culture plate. The cultures are incubated aerobically (static culture) at 37°C for 16-20 hours . 4 week old mice (female,18g - 22g, strain MF1) are each infected by subcutaneous injection of 0.5ml. of this broth culture of *Staphylococcus aureus* WCUH29 (diluted in broth to approximately $10^8$ cfu/ml.) into the anterior , right lower quadrant (groin area). Mice should be monitored regularly during the first 24 hours after infection, then daily until termination of study. Animals with signs of systemic infection, i.e. lethargy, ruffled appearance, isolation from group, should be monitored closely and if signs progress to moribundancy, the animal should be culled immediately.
Visible external signs of lesion development will be seen 24-48h after infection. Examination of the abdomen of the animal will show the raised outline of the abscess beneath the skin. The localised lesion should remain in the right lower quadrant, but may occasionally spread to the left lower quadrant, and superiorly to the thorax. On occasions, the abscess may rupture through the overlying skin layers. In such cases the affected animal should be culled immediately and the tissues sampled if possible. Failure to cull the animal may result in the necrotic skin tissue overlying the abscess being sloughed off, exposing the abdominal muscle wall.
Approximately 96 hours after infection, animals are killed using carbon dioxide asphyxiation. To minimise delay between death and tissue processing /storage, mice should be killed individually rather than in groups.The dead animal is placed onto its back and the fur swabbed liberally with 70% alcohol. An initial incision using scissors is made through the skin of the abdominal left lower quadrant, travelling superiorly up to, then across the thorax. The incision is completed by cutting inferiorly to the abdominal lower right quadrant. Care should be taken not to penetrate the abdominal wall. Holding the skin flap with forceps, the skin is gently pulled way from the abdomen. The exposed abscess, which covers the peritoneal wall but generally does not penetrate the muscle sheet completely, is excised, taking care not to puncture the viscera

The abscess/muscle sheet and other infected tissue may require cutting in sections, prior to flash-freezing in liquid nitrogen, thereby allowing easier storage in plastic collecting vials.

**b) Pyelonephritis Model: Isolation of tissue infected with *Staphylococcus aureus* WCUH29 from a murine model of hematogenous pyelonephritis.**

Overnight cultures of S. *aureus* WCUH29 are started from single colonies in 5 ml of tryptic soy broth (TSB) and grown at 37°C with shaking. The cultures are then washed twice in sterile phosphate-buffered saline (PBS) and diluted to an $A_{600}$ = 0.3. Male CD-1 mice (18 - 20g) are infected with 0.2 ml of this suspension by tail vein inoculation using a 30g needle attached to a tuberculin syringe. Each mouse receives approximately $4 \times 10^7$ bacteria in this fashion. Mice are monitored daily for signs of illness, and usually within 48 hours show signs of lethargy, ruffled fur, sluggishness; animals which appear moribund are euthanized prior to the end of the experiment. All animals are euthanized via carbon dioxide overdose seven days post-infection. The animal is placed on its back and swabbed with ethanol, and then with RNAZap, and instruments are swabbed as well. The abdominal cavity is opened and the kidneys aseptically removed, cut into four pieces, and placed in cryovials which are immediately frozen in liquid nitrogen.

**c) Isolation of *Staphylococcus aureus* WCUH29 RNA from infected tissue samples**

4-6 infected tissue samples(each approx 0.5-0.7g) in 2ml screw-cap tubes are removed from -80°C.storage into a dry ice ethanol bath In a microbiological safety cabinet the samples are disrupted individually whilst the remaining samples are kept cold in the dry ice ethanol bath. To disrupt the bacteria within the tissue sample 1ml of TRIzol Reagent (Gibco BRL, Life Technologies) is added followed by enough 0.1mm zirconia/silica beads to almost fill the tube, the lid is replaced taking care not to get any beads into the screw thread so as to ensure a good seal and eliminate aerosol generation. The sample is then homogenised in a Mini-BeadBeater Type BX-4 (Biospec Products). Necrotic fatty tissue isstrain treated for 100 seconds at 5000 rpm in order to achieve bacterial lysis. *In vivo* grown bacteria require longer treatment than *in vitro* grown Staphylococcus aureus Staphylococcus which are disrupted by a 30 second bead-beat.

After bead-beating the tubes are chilled on ice before opening in a fume-hood as heat generated during disruption may degrade the TRIzol and release cyanide.

200 microlitres of chloroform is then added and the tubes shaken by hand for 15 seconds to ensure complete mixing. After 2-3 minutes at room temperature the tubes are spun down at 12,000 x g, 4°C for 15minutes and RNA extraction is then continued according to the method given by the manufacturers of TRIzol Reagent i.e.:- The aqueous phase, approx 0.6 ml, is transferred to a sterile eppendorf tube and 0.5 ml of isopropanol is added. After 10 minutes at room temperature the samples are spun at 12,000 x g, 4°C for 10 minutes. The supernatant is removed and discarded then the RNA pellet is washed with 1 ml 75% ethanol. A brief vortex is used to mix the sample before centrifuging at 7,500 x g, 4 °C for 5 minutes. The ethanol is removed and the RNA pellet dried under vacuum for no more than 5 minutes. Samples are then resuspended by repeated pipetting in 100 microlitres of DEPC treated water, followed by 5-10 minutes at 55 °C. Finally, after at least 1 minute on ice, 200 units of Rnasin (Promega) is added.

RNA preparations are stored at -80 °C for up to one month. For longer term storage the RNA precipitate can be stored at the wash stage of the protocol in 75% ethanol for at least one year at -20 °C.

Quality of the RNA isolated is assessed by running samples on 1% agarose gels. 1 x TBE gels stained with ethidium bromide are used to visualise total RNA yields. To demonstrate the isolation of bacterial RNA from the infected tissue 1 x MOPS, 2.2M formaldehyde gels are run and vacuum blotted to Hybond-N (Amersham). The blot is then hybridised with a $^{32}$P labelled oligonucletide probe specific to 16s rRNA of *Staphylococcus aureus* (K.Greisen, M. Loeffelholz, A. Purohit and D. Leong. J.Clin. (1994) Microbiol. 32 335-351 ). An oligonucleotide derived from a sequence of the invention is used as a probe. The size of the hybridising band is compared to that of control RNA isolated from *in vitro* grown *Staphylococcus aureus* WCUH29 in the Northern blot. Correct sized bacterial 16s rRNA bands can be detected in total RNA samples which show extensive degradation of the mammalian RNA when visualised on TBE gels.

**d) The removal of DNA from *Staphylococcus aureus* WCUH29-derived RNA**

DNA was removed from 73 microlitre samples of RNA by a 15 minute treatment on ice with 3 units of DNAaseI, amplification grade (Gibco BRL, Life Technologies) in the buffer supplied with the addition of 200 units of Rnasin (Promega) in a final volume of 90 microlitres.

The DNAase was inactivated and removed by treatment with TRIzol LS Reagent (Gibco BRL, Life Technologies) according to the manufacturers protocol.

DNAase treated RNA was resuspended in 73 microlitres of DEPC treated water with the addition of Rnasin as described in Method 1.

### e) The preparation of cDNA from RNA samples derived from infected tissue

10 microlitre samples of DNAase treated RNA are reverse transcribed using a SuperScript Preamplification System for First Strand cDNA Synthesis kit (Gibco BRL, Life Technologies) according to the manufacturers instructions. 1 nanogram of random hexamers is used to prime each reaction. Controls without the addition of SuperScriptII reverse transcriptase are also run. Both +/-RT samples are treated with RNaseH before proceeding to the PCR reaction

### f) The use of PCR to determine the presence of a bacterial cDNA species

PCR reactions are set up on ice in 0.2ml tubes by adding the following components: 45 microlitres PCR SUPERMIX (Gibco BRL, Life Technologies); 1 microlitre 50mM MgCl$_2$, to adjust final concentration to 2.5mM; 1 microlitre PCR primers(optimally 18-25 basepairs in length and designed to possess similar annealing temperatures), each primer at 10mM initial concentration; and 2 microlitres cDNA.

PCR reactions are run on a Perkin Elmer GeneAmp PCR System 9600 as follows: 5 minutes at 95 °C, then 50 cycles of 30 seconds each at 94 °C, 42 °C and 72 °C followed by 3 minutes at 72 °C and then a hold temperature of 4 °C. (the number of cycles is optimally 30-50 to determine the appearance or lack of a PCR product and optimally 8-30 cycles if an estimation of the starting quantity of cDNA from the RT reaction is to be made); 10 microlitre aliquots are then run out on 1% 1 x TBE gels stained with ethidium bromide with PCR product, if present, sizes estimated by comparison to a 100 bp DNA Ladder (Gibco BRL, Life Technologies). Alternatively if the PCR products are conveniently labelled by the use of a labelled PCR primer (e.g. labelled at the 5'end with a dye) a suitable aliquot of the PCR product is run out on a polyacrylamide sequencing gel and its presence and quantity detected using a suitable gel scanning system (e.g. ABI Prism™ 377 Sequencer using GeneScan™ software as supplied by Perkin Elmer).

RT/PCR controls may include +/- reverse transcriptase reactions, 16s rRNA primers or DNA specific primer pairs designed to produce PCR products from non-transcribed *Staphylococcus aureus* WCUH29 genomic sequences.

To test the efficiency of the primer pairs they are used in DNA PCR with *Staphylococcus aureus* WCUH29 total DNA. PCR reactions are set up and run as described above using approx. 1 microgram of DNA in place of the cDNA and 35 cycles of PCR.

Primer pairs which fail to give the predicted sized product in either DNA PCR or RT/PCR are PCR failures and as such are uninformative. Of those which give the correct size product with DNA PCR two classes are distinguished in RT/PCR: 1.Genes which are not transcribed *in vivo* reproducibly fail to give a product in RT/PCR; and 2.Genes which are transcribed *in vivo* reproducibly give the correct size product in RT/PCR and show a stronger signal in the +RT samples than the signal (if at all present) in -RT controls.

Using the method described in this example and primer pairs set forth in the Sequence Listing (see Table 1 for further details), each of the the polynucleotides in the Sequence Listing were demonstrated to be *in vivo* expressed.

## Claims

1. An isolated polynucleotide comprising a polynucleotide sequence selected from the group consisting of:

    (a) a polynucleotide having at least a 70% identity to a polynucleotide encoding a polypeptide comprising the amino acid sequence of SEQ ID NO: 11, 12, 13, 14, 15 or 20;
    (b) a polynucleotide having at least a 70% identity to a polynucleotide encoding the same mature polypeptide expressed by the gene contained in the *Staphylococcus aureus* of the deposited strain that was sequenced to obtain a polynucleotide sequence of SEQ ID NO:1, 2, 3, 4, 5 or 10;
    (c) a polynucleotide encoding a polypeptide comprising an amino acid sequence which is at least 70% identical to the amino acid sequence of SEQ ID NO: 11, 12, 13, 14, 15 or 20;
    (d) a polynucleotide which is complementary to the polynucleotide of (a), (b) or (c); and

(e) a polynucleotide comprising at least 15 sequential bases of the polynucleotide of (a), (b), (c) or (d).

2. The polynucleotide of Claim 1 wherein the polynucleotide is DNA.

3. The polynucleotide of Claim 1 wherein the polynucleotide is RNA.

4. The polynucleotide of Claim 2 comprising the nucleic acid sequence selected from the group consisting of the nucleic acid sequences set forth in SEQ ID NO:1, 2, 3, 4, 5 and 10.

5. The polynucleotide of Claim 2 which encodes a polypeptide comprising an amino acid sequence selected from the group consisting of the amino acid sequences set forth in SEQ ID NO: 11, 12, 13, 14, 15 and 20.

6. A vector comprising the polynucleotide of Claim 1.

7. A host cell comprising the vector of Claim 6.

8. A process for producing a polypeptide comprising: expressing from the host cell of Claim 7 a polypeptide encoded by said DNA.

9. A process for producing a polypeptide or fragment comprising culturing a host of claim 7 under conditions sufficient for the production of said polypeptide or fragment.

10. A polypeptide comprising an amino acid sequence which is at least 70% identical to an amino acid sequence selected from the group consisting of the amino acid sequences set forth in SEQ ID NO: 11, 12, 13, 14, 15 and 20.

11. A polypeptide comprising an amino acid sequence selected from the group consisting of the amino acid sequences set forth in SEQ ID NO: 11, 12, 13, 14, 15 and 20

12. An antibody against the polypeptide of claim 10.

13. An antagonist or agonist of the activity or expression of the polypeptide of claim 10.

14. A method for the treatment or prevention of disease of an individual comprising: administering to the individual a therapeutically effective amount of the polypeptide of claim 10.

15. A method for the treatment of an individual having need to inhibit a bacterial polypeptide comprising: administering to the individual a therapeutically effective amount of the antagonist of Claim 13.

16. A process for diagnosing a disease related to expression or activity of the polypeptide of claim 10 in an individual comprising:

(a) determining a nucleic acid sequence encoding said polypeptide, and/or
(b) analyzing for the presence or amount of said polypeptide in a sample derived from the individual.

17. A method for identifying compounds which interact with and inhibit or activate an activity of the polypeptide of claim 10 comprising:

contacting a composition comprising the polypeptide with the compound to be screened under conditions to permit interaction between the compound and the polypeptide to assess the interaction of a compound, such interaction being associated with a second component capable of providing a detectable signal in response to the interaction of the polypeptide with the compound;
and determining whether the compound interacts with and activates or inhibits an activity of the polypeptide by detecting the presence or absence of a signal generated from the interaction of the compound with the polypeptide.

18. A method for inducing an immunological response in a mammal which comprises inoculating the mammal with the polypeptide of claim 10, or a fragment or variant thereof, adequate to produce antibody and/or T cell immune response to protect said animal from disease.

**19.** A polynucleotide selected from the group consisting of SEQ ID NO:1, 2, 3, 4, 5 and 10.

**20.** A polypeptide selected from the group consisting of SEQ ID NO: 11, 12, 13, 14, 15 and 20.

**21.** An isolated polynucleotide comprising a polynucleotide sequence selected from the group consisting of:

(a) a polynucleotide having at least a 70% identity to a polynucleotide encoding a polypeptide comprising the amino acid sequence of SEQ ID NO: 16, 17, 18, 19, 45, 46, 47 or 48;
(b) a polynucleotide having at least a 70% identity to a polynucleotide encoding the same mature polypeptide expressed by the gene contained in the *Staphylococcus aureus* of the deposited strain that was sequenced to obtain a polynucleotide sequence of SEQ ID NO:6, 7, 8, 9, 41, 42, 43 or 44;
(c) a polynucleotide encoding a polypeptide comprising an amino acid sequence which is at least 70% identical to the amino acid sequence of SEQ ID NO:16, 17, 18, 19, 45, 46, 47 or 48;
(d) a polynucleotide which is complementary to the polynucleotide of (a), (b) or (c); and
(e) a polynucleotide comprising at least 15 sequential bases of the polynucleotide of (a), (b), (c) or (d).

**22.** The polynucleotide of Claim 21 wherein the polynucleotide is DNA.

**23.** The polynucleotide of Claim 21 wherein the polynucleotide is RNA.

**24.** The polynucleotide of Claim 22 comprising the nucleic acid sequence selected from the group consisting of the nucleic acid sequences set forth in SEQ ID NO:6, 7, 8, 9, 41, 42, 43 and 44.

**25.** The polynucleotide of Claim 22 which encodes a polypeptide comprising an amino acid sequence selected from the group consisting of the amino acid sequences set forth in SEQ ID NO:16, 17, 18, 19, 45, 46, 47 and 48.

**26.** A vector comprising the polynucleotide of Claim 21.

**27.** A host cell comprising the vector of Claim 26.

**28.** A process for producing a polypeptide comprising: expressing from the host cell of Claim 27 a polypeptide encoded by said DNA.

**29.** A process for producing a polypeptide or fragment comprising culturing a host of claim 27 under conditions sufficient for the production of said polypeptide or fragment.

**30.** A polypeptide comprising an amino acid sequence which is at least 70% identical to an amino acid sequence selected from the group consisting of the amino acid sequences set forth in the SEQ ID NO:16, 17, 18, 19, 45, 46, 47 and 48.

**31.** A polypeptide comprising an amino acid sequence selected from the group consisting of the amino acid sequences set forth in the SEQ ID NO:16, 17, 18, 19, 45, 46, 47 and 48.

**32.** An antibody against the polypeptide of claim 30.

**33.** An antagonist or agonist of the activity or expression of the polypeptide of claim 30.

**34.** A method for the treatment or prevention of disease of an individual comprising: administering to the individual a therapeutically effective amount of the polypeptide of claim 30.

**35.** A method for the treatment of an individual having need to inhibit a bacterial polypeptide comprising: administering to the individual a therapeutically effective amount of the antagonist of Claim 33.

**36.** A process for diagnosing a disease related to expression or activity of the polypeptide of claim 30 in an individual comprising:

(a) determining a nucleic acid sequence encoding said polypeptide, and/or
(b) analyzing for the presence or amount of said polypeptide in a sample derived from the individual.

37. A method for identifying compounds which interact with and inhibit or activate an activity of the polypeptide of claim 30 comprising:

contacting a composition comprising the polypeptide with the compound to be screened under conditions to permit interaction between the compound and the polypeptide to assess the interaction of a compound, such interaction being associated with a second component capable of providing a detectable signal in response to the interaction of the polypeptide with the compound;
and determining whether the compound interacts with and activates or inhibits an activity of the polypeptide by detecting the presence or absence of a signal generated from the interaction of the compound with the polypeptide.

38. A method for inducing an immunological response in a mammal which comprises inoculating the mammal with the polypeptide of claim 30, or a fragment or variant thereof, adequate to produce antibody and/or T cell immune response to protect said animal from disease.

39. A polynucleotide selected from the group consisting of SEQ ID NO:6, 7, 8, 9, 41, 42, 43 and 44.

40. A polypeptide selected from the group consisting of SEQ ID NO:16, 17, 18, 19, 45, 46, 47 and 48.